# EUROPEAN PATENT APPLICATION

(11) **EP 2 708 243 A1**
(43) Date of publication of application: **19.03.2014**
(21) Application number: 12006502.4
(22) Date of filing: 17.09.2012
(51) Int. Cl.: A61K 47/48, A61P 35/00

(54) **Receptor ligand linked cytotoxic molecules**

(71) Applicant: OntoChem GmbH, 06120 Halle / Saale (DE)
(72) Inventor: Weber, Lutz, 82110 Germering (DE)
(74) Representative: Forstmeyer, Dietmar

(57) **Abstract**

The present invention relates to conjugates comprising cytotoxic compounds conjugated via a linker moiety to neuropeptide Y1 receptor ligands and their use for the treatment of cancer and other diseases.

## Description

The present invention refers to novel cytotoxic molecules and their use for the treatment of cancer and other diseases.

It is one object of the present invention to provide novel cytotoxic molecules which link to a receptor ligand and have an activity against cancer cells and tuneable pharmacological properties especially for efficiently targeting those cancer cells that express the neuropeptide Y receptor 1. In particular, the present invention describes receptor ligand linked cytotoxic compounds that are derivatives of a cytotoxic compound, conjugated via a linker moiety to a peptide that is a ligand of the neuropeptide Y receptor 1, providing thereby the targeting property of the compounds to enhance the selectivity and the therapeutic window of the cytotoxic compounds.

A variety of cytotoxic compounds are known, for example naturally occurring tubulysins or derivatives thereof and others (A. Dömling, W. Richter, Mol. Diversity 2005, 9, 141-147) that exhibit highly potent cytotoxic activity against a large panel of cancer cell lines. However, the use of those compounds as novel treatments for cancer is limited due to their small therapeutic window and insufficient selectivity against non-cancer cells. One known strategy to increase the therapeutic window of highly potent cytotoxics is to conjugate those molecules to cancer specific ligands such as antibodies (US 2010/0092496 and US 2011/0166319), peptides (US 2011/0166319) or small molecule ligands of receptors such as for example the folate receptor (US 2011/0027274 and 2011/0172254) or molecules that are internalized in vivo such as for example vitamins (US 2010/0004276).

While this general principle is known, it is not possible to predict which specific compound will be a useful cytotoxic conjugate as many factors influence the potential therapeutic use of such conjugates. In general, a useful conjugate must exhibit 4 major properties that are all required for the selective and potent killing of cancer cells:
1. Sufficient half-life in vivo to reach the cancer cells;
2. selective binding to cancer cells;
3. efficient internalization of the conjugate into the cell; and
4. cleavage of the cytotoxic molecule from the conjugate within the cell, efficient killing of tumor cells.

These properties are difficult to combine in one molecule. For example, the conjugation may significantly decrease both the affinity and/or selectivity or other binding properties of the cancer specific ligand towards its target. Internalization of the cytotoxic drug conjugate may happen through an unspecific transport into the cell such as for example endocytosis, decreasing thereby the therapeutic window. The specific internalization of the ligand through for example receptor activation and subsequent internalization may be abolished by the conjugation. The cleavage of the cytotoxic molecule from the targeting ligand may already happen in the extracytolic space or in the blood, resulting in a general toxicity of the conjugate. Alternatively, the cleavage within the cell maybe slower as required or results in a more inactive toxin by having a part of the linker still attached or may not happen at all.

It has been shown that the NPY-1 receptor is overexpressed in certain cancer types such as breast cancer and especially metastatic breast cancer, but also in other cancer conditions such as renal cell carcinomas, gastrointestinal stromal tumors, nephroblastomas, neuroblastic tumors, paragangliomas, pheochromocytomas, adrenal cortical tumors, ovarian sex cord-stromal tumors, and ovarian adeno carcinomas (Körner and Reubi, Peptides 28, 419-425 (2007)). Interestingly, the NPY 1 receptor, upon activation by its natural ligand, the NPY peptide, internalizes.

Therefore, it is interesting to consider the NPY-1 receptor for targeting. This approach has been published by Zwanziger (Zwanziger et al. Bioconjugate Chem. 2008, 19, 1430-1438), using a chelator bound to modified NPY peptides for diagnostic purposes. The half-life of some conjugates was found to be much longer (T1/2 > 24 h) when compared with the native peptide (T1/2 ∼ 4 minutes) in different tissues. For example, the modified NPY included a change in position 7 to phenylalanine and in position 34 to proline. This modified NPY molecule was claimed to be especially selective for the NPY 1 receptor in comparison to the competing NPY-2, -4, -5 or -6 receptors. While these results were promising, the conjugates had a very low uptake by the tumors, satisfying prerequisite 1 and 2, but violating prerequisite 3 from above.

NPY-1 ligand toxin conjugates have been published by Langer (Langer et al. J. Med. Chem. 2001, 44, 1341-1348), using daunorubicin and doxorubicin as cytotoxic drugs, conjugated to the native NPY peptide. The different conjugates were able to bind to the receptor with affinities ranging from 25 to 51 nM, but only the compound containing the acid-sensitive hydrazone linked Daunorubicin showed cytotoxic activity comparable to the free daunorubicin. However, since the hydrazone linker is already cleaved in serum, this does not satisfy prerequisite 1 and 4 from above and such conjugates cannot be used as therapeutics. These examples are illustrating the problems that need to be solved when new targeted cytotoxic conjugates with therapeutic utility shall be invented.

It is the aim of the present invention to provide novel compounds and methods that satisfy all 4 prerequisites for targeted drug conjugates and that utilize NPY-1 receptor specific ligands coupled via suitable strategies to cytotoxic molecules.

The present invention provides a compound of Formula (I):

**A-L-Pep** (I)

wherein

**A** is a suitable (preferably highly potent) molecule (especially toxic, e.g. a cytotoxic molecule), preferably with a cellular activity below 100 nanomolar concentration and preferably, A has the ability to kill cancer cells e.g. by cytostatic, cytotoxic, antiangiogenic or other properties.

L is a releasable or non-releasable linker or a bond between A and Pep, and

Pep is a NPY-1 receptor binding ligand, exhibiting additionally NPY-1 receptor activating and internalizing properties;
or a pharmacologically acceptable salt, a solvate, a hydrate or a pharmacologically acceptable formulation thereof.

Preferably, A is a cytotoxic or cytostatic molecule especially selected from highly potent molecules like tubulysins and derivatives thereof, natural and synthetic epothilones and derivatives thereof, auristatins, dolastatins, natural and synthetic vincristine and its analogues, natural and synthetic vinblastine and its analogues, amanitine and its analogues, maytansines and its analogues, taxanes, Nemorubicin, PNU-159682, pyrrolobenzodiazepins and dimers, duocarmycins and its analogues.

Preferably the present invention provides compounds of Formula (II): 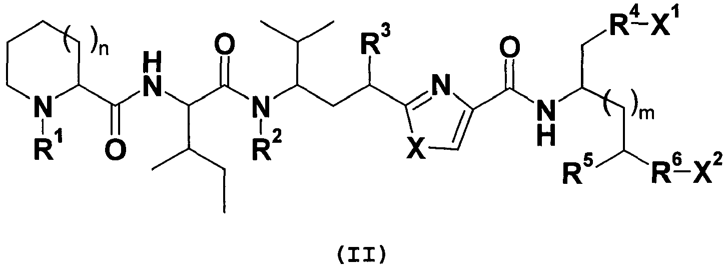 wherein
n = 0, 1, 2;
R¹ is an alkyl or a heteroalkyl group;
R² is hydrogen, an optionally substituted alkyl, alkenyl, alkinyl, heteroalkyl, cycloalkyl, heterocycloalkyl, alkylcycloalkyl, heteroalkylcycloalkyl, aryl, heteroaryl, aralkyl or heteroaralkyl group;
R³ is is hydrogen, OH, an optionally substituted alkyl, alkenyl, alkinyl, heteroalkyl, cycloalkyl, heterocycloalkyl, alkylcycloalkyl, heteroalkylcycloalkyl, aryl, heteroaryl, aralkyl or heteroaralkyl group;
X is O or S (especially S);
R⁴ is an optionally substituted arylene, heteroarylene, heterocycloalkylene, heteroalkylcycloalkylene, aralkylene or heteroaralkylene group;
R⁵ is hydrogen, an optionally substituted C₁-C₆ alkyl group, or an optionally substituted aryl or heteroaryl group;
R⁶ is -CO₂- -CONH-, -CO-, -CONHNH-, -O-, -NH-, -S-, -SO-, -SO₂-, -OP(=O)O₂- or a branched or unbranched substituted or unsubstituted alkylene, cycloalkylene, heteroalkylene or heterocycloalkylene group;
m is 0, 1, 2 or 3;
X¹ is -L-Pep and X² is H or X¹ is H and X² is -L-Pep; and
L and Pep are as defined herein;
or a pharmacologically acceptable salt, a solvate, a hydrate or a pharmacologically acceptable formulation thereof.

Preferably R² is optionally branched alkyl like methyl, ethyl, propyl, isopropyl, n-butyl, isobutyl, CH₂OR⁷, C(O)R⁷ or CH₂OCOR⁸, wherein R⁷ is optionally branched alkyl, especially isopropyl, R⁸ is optionally branched C₁-C₆ alkyl or C₂-C₆ alkenyl.

Further preferred R² is an optionally substituted alkyl; -R⁹-OR¹⁰, wherein R⁹ is alkylen (especially methylen or ethylen), and R¹⁰ is an alkyl, alkenyl, cycloalkyl, heteroalkyl, heterocycloalkyl, aryl, or heteroaryl group; C(O)R⁷, wherein R⁷ is an alkyl group, especially isopropyl; CH₂OCOPh; CH₂OCOCH₂Ph; -R⁹-NR¹¹R¹², wherein R⁹ is alkylen, and R¹¹ and R¹² are independently of each other H or alkyl; or R¹³-OH, wherein R¹³ is alkylen; or CH₂OCOR⁸, wherein R⁸ is optionally branched C₁-C₆ alkyl or C₂-C₆ alkenyl.

More preferred R² is a C₁-C₈-alkyl; -R⁹-O-R¹⁰, wherein R⁹ is C₁-C₆-alkylen, especially methylen or ethylen, and R¹⁰ is a C₁-C₆-alkyl, C₂-C₆-alkenyl, cycloalkyl, heteroalkyl, aryl, or heteroaryl group; C(O)R ⁷, wherein R⁷ is a C₁-C₆-alkyl group, especially isopropyl; CH₂OCOPh; or CH₂OCOCH₂Ph.

Preferably R³ is H, -O(C=O)-(C₁₋₄)alkyl, O-alkyl or O-acetyl.

Preferably, when X¹ is hydrogen, R⁴ is aralkyl or heteroaralkyl; especially preferred R⁴ is a group of the Formula 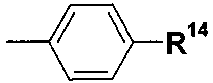 wherein R¹⁴ is H, halogen, OH, NO₂, NH₂, CN, alkyl, heteroalkyl, cyclo-alkyl, heterocycloalkyl, aryl, heteroaryl, alkyloxy, alkyl amino or dialkyl amino.

Especially preferred, R¹⁴ is H, OH, halogen, NH₂, alkyloxy, phenyl, alkyl amino or dialkyl amino.

Preferably m is 0 or 1.

Further preferably, X¹ is hydrogen and X² is -L-Pep.

Especially preferred are compounds of the following formula (III) : 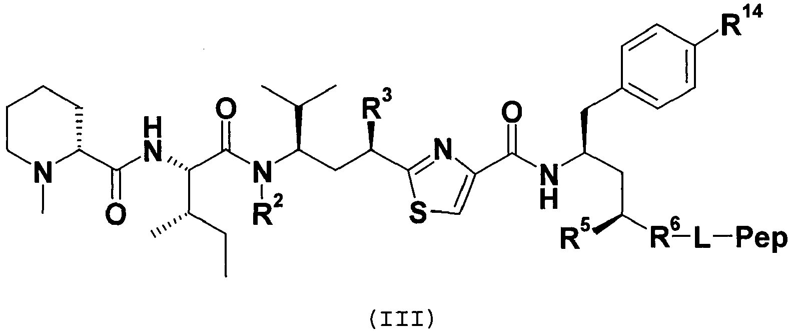 wherein
R² is C₁-C₆ alkyl (especially CH₃, ethyl, propyl, isopropyl, isobutyl, n-pentyl or n-hexyl), CH₂Ph, CH₂OCH₂CH(CH₃)₂, CH₂OCOCHC₁-C₆-Alkyl, CH₂OCOCH₂Ph or CH₂OCOPh;
R³ is H, OAc or OPr;
R⁵ is CH₃ or H;
R⁶ is -COO-, -CONH-, -CONHNH-, -O-, -NH-, -CH₂O- -CH₂NH- or -CH₂S-;
R¹⁴ is H, F, OH, NH₂, CH₃, OMe or Ph; and
L and Pep are as defined above;
or a pharmacologically acceptable salt, a solvate, a hydrate or a pharmacologically acceptable formulation thereof.

Further preferably, A is a compound of Formula (Tub) 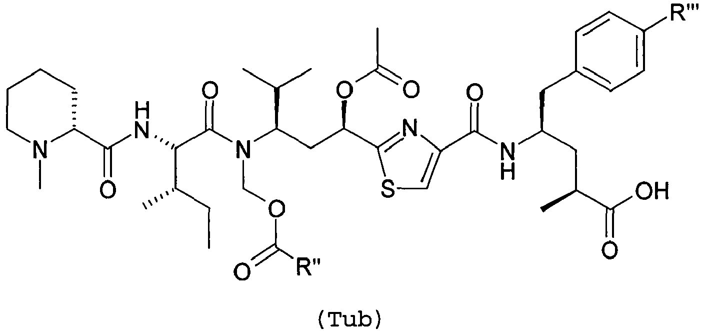 wherein R" is H, alkyl, alkenyl, aryl, or heteroaryl and R''' is H, OH, and wherein one hydrogen atom of the compound of Formula (Tub) is replaced by the bond to linker L.

Further preferred, A is selected from the following compounds: Tubulysin A, B, C, D, E, F, G, H and I, wherein one hydrogen atom is replaced by the bond to linker L.

Further preferably, A is a compound of formula (IV), wherein one hydrogen atom is replaced by the bond to linker L: 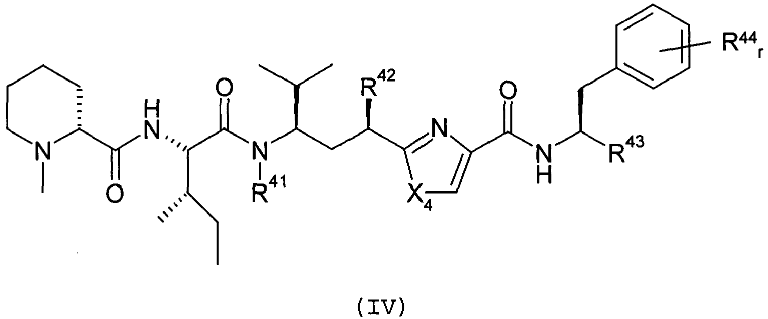 wherein
R⁴¹ is H or an alkyl, alkenyl, alkynyl, CO-alkyl, heteroalkyl, aralkyl or heteroaralkyl group, all of which may optionally be substituted;
R⁴² is OH, an alkyl, alkenyl, alkynyl, -O-alkyl, -O-alkenyl, - O-alkynyl, -O-CO-alkyl or heteroalkyl group, all of which may optionally be substituted;
R⁴³ is a group of formula CO₂H, CO₂R⁴⁵, CONHR⁴⁵, CONR₂⁴⁵, or a group of formula CH₂OH or 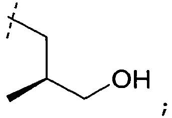 R⁴⁵ independently being an alkyl, aryl, aralkyl or heteroalkyl group;
X₄ is S or O;
R⁴⁴ is independently optionally substituted alkyl (e.g. methyl), optionally substituted heteroalkyl (e.g. OMe), halogen, CN, NO₂ or OH; and r is 0, 1, 2, 3, 4 or 5.

Preferably, R⁴¹ is H or C₁-C₆ alkyl; especially a methyl, ethyl, propyl, butyl, isopropyl, isobutyl, n-pentyl or n-hexyl.

Preferably, R⁴² is -O-CO-alkyl, alkyl or heteroalkyl (e.g. -O-alkyl, -O-alkyl-O-alkyl), especially O-Acetyl (OAc, OCOCH₃), -O-propyl or -OCH₂OCH₃.

Preferably, R⁴³ is a group of formula CO₂H or CO₂R⁴⁵, with R⁴⁵ preferably being an alkyl group.

Further preferably, R⁴³ is 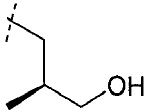 or -CH₂OH.

Preferably X₄ is S.

Preferably R⁴⁴ is independently optionally substituted alkyl, halogen (e.g. F or Cl) or OH.

Preferably r is 0, 1, 2 or 3, especially 0 or 1.

Especially preferred are compounds of formula (IV) wherein: R⁴¹ is C₁-C₆ alkyl, especially -CH₃, ethyl, propyl, butyl, isopropyl, isobutyl, n-pentyl, n-hexyl; R⁴² is -O-CO-alkyl or heteroalkyl, especially -O-CO-CH₃, -O-propyl or -OCH₂OCH₃;

R⁴³ is -CO₂H, -CO₂R⁴⁵, -CH₂OH or 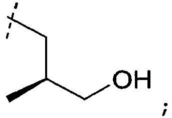 R⁴⁵ is an alkyl group;
X₄ is S;
R⁴⁴ is independently optionally substituted alkyl, halogen (e.g. F or Cl) or OH; and
r is 0 or 1.

Further preferably, A is a compound of Formula (Epo) 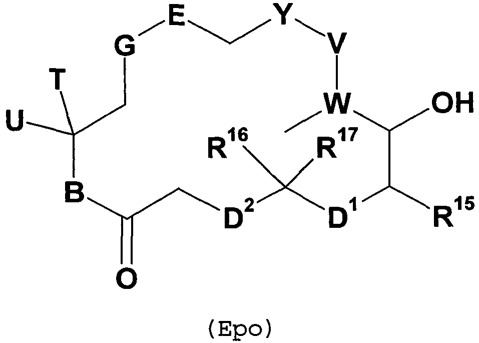 wherein
T is a heteroalkyl-, heterocycloalkyl-, heteroalkylcycloalkyl-, heteroaryl- or heteroarylalkyl-group,
U is hydrogen, halogen, an alkyl, heteroalkyl-, heterocycloalkyl-, heteroalkylcycloalkyl-, heteroaryl- or heteroarylalkyl-group,
G-E is selected from the following groups, 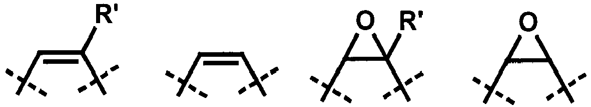 wherein R' is F or a C₁-C₃ alkyl group or G-E is part of an optionally substituted phenyl ring,
R¹⁵ is a C₁-C₄-alkyl-, a C₁-C₄-alkenyl-, a C₁-C₄-alkynyl- or a C₃-C₄-cycloalkyl-group,
Y-V-W is a group of formula CH=CH-CH, CH₂-CH₂-CH or CH₂-CH=C, wherein the double bonds give rise to cis or trans isomers,
D¹ is C=O or S(=O)p, wherein p is 0, 1 or 2,
D² is CHOH, O or S(=O)q, wherein q is 0, 1 or 2,
B is oxygen or a group of the formula NR¹⁸, wherein R¹⁸ is hydrogen, an alkyl-, alkenyl-, alkynyl-, heteroalkyl-, aryl-, heteroaryl-, cycloalkyl-, alkylcycloalkyl-, heteroalkylcycloalkyl-, heterocycloalkyl-, aralkyl- or heteroarylalkyl-group and
R¹⁶ and R¹⁷ independently from each other represent hydrogen, C₁-C₄-alkyl or together are part of a cycloalkyl group with 3 or 4 ring atoms,
wherein one hydrogen atom of the compound of Formula (Epo) is replaced by the bond to linker L.

Preferred are compounds of formula (Epo), wherein T is a group of the formula -C(CH₃)=CHR¹⁹ or -CH=CHR¹⁹, wherein R¹⁹ is a heteroaryl- or a heteroarylalkyl group.

Further preferred are compounds of formula (Epo), wherein T is a group of formula (EpoI) or (EpoII) (especially a group (EpoI)): 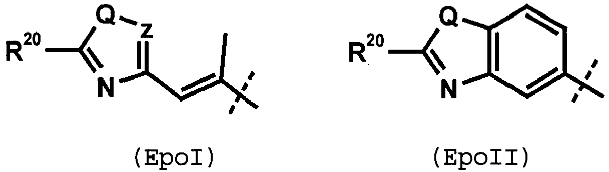 wherein Q is sulphur, oxygen or NR²¹ (especially oxygen or sulphur), wherein R²¹ is hydrogen, C₁-C₄ alkyl or C₁-C₄ heteroalkyl, z is Nitrogen or CH (especially CH) and R²⁰ is OR²², NHR²², C₁-C₄ alkyl, C₁-C₄ alkenyl, C₁-C₄ alkynyl or C₁-C₆ heteroalkyl (especially methyl, CH₂OR²² or CH₂NHR²²), wherein R²² is hydrogen, C₁-C₄ alkyl or C₁-C₄ heteroalkyl (especially hydrogen).

Further preferably, D¹ is C=O and D² is CHOH or S(=O)q, wherein q is 0, 1 or 2.

Moreover preferably, D¹ is S(=O)ₚ, wherein p is 0, 1 or 2 and D² is CHOH.

Moreover preferred are compounds of formula (Epo), wherein R¹⁸ is hydrogen or C₁-C₄ alkyl.

Further preferred are compounds of formula (Epo), wherein B is oxygen or NH (especially oxygen).

Moreover preferred are compounds of formula (Epo), wherein R¹⁵ is methyl or ethyl (especially methyl).

Further preferred are compounds of formula (Epo), wherein R¹⁶ and R¹⁷ are methyl groups.

Moreover preferred are compounds of formula (Epo), wherein U is hydrogen, fluorine, methyl, trifluoromethyl or COOH (especially hydrogen).

Further preferred are compounds of formula (Epo), wherein the absolute stereochemistry is the same as in the natural occurring epothilones B and/or D.

Moreover preferred are compounds of formula (Epo), wherein R' is CH₃ or CF₃.

Further preferred are compounds of formula (Epo), wherein D is C=O.

Preferably, L is a bond or an alkylene, alkenylene, alkinylene, heteroalkylene, arylene, heteroarylene, cycloalkylene, heterocycloalkylene, alkylcycloalkylene, heteroalkylcycloalkylene, aralkylene or heteroaralkylene group.

Further preferably, L is a bond or a heteroalkylene, a heteroalkylcycloalkylene or a heteroaralkylene group (i.e. preferably a group comprising a heteroalkylene group).

In a preferred embodiment, L is a chain of atoms selected from C, N, O, S, Si, and P that covalently connects A to Pep. L may have a wide variety of lengths, such as in the range from about 2 to about 100 atoms. The atoms used in forming L may be combined in all chemically relevant ways, such as chains of carbon atoms forming alkylene, alkenylene, and alkynylene groups, and the like; chains of carbon and oxygen atoms forming ethers, polyoxyalkylene groups, or when combined with carbonyl groups forming esters and carbonates, and the like; chains of carbon and nitrogen atoms forming amines, imines, polyamines, hydrazines, hydrazones, or when combined with carbonyl groups forming amides, ureas, semicarbazides, carbazides, and the like; chains of carbon, nitrogen, and oxygen atoms forming alkoxyamines, alkoxylamines, or when combined with carbonyl groups forming urethanes, amino acids, acyloxylamines, hydroxamic acids, and the like; and many others. In addition, it is to be understood that the atoms forming the chain in each of the foregoing illustrative embodiments may be either saturated or unsaturated, such that for example, alkanes, alkenes, alkynes, imines, and the like may be radicals that are included in L. In addition, it is to be understood that the atoms forming L may also be cyclized upon each other to form divalent cyclic structures that form the linker, including cyclo alkanes, cyclic ethers, cyclic amines, arylenes, heteroarylenes, and the like in the linker.

Further preferably, L includes radicals that form at least one releasable linker, and optionally one or more spacer linkers. As used herein, the term releasable linker refers to a linker that includes at least one bond that can be broken under physiological conditions, such as a pH-labile, acid-labile, base-labile, oxidatively labile, metabolically labile, biochemically labile, or enzyme-labile bond. It is appreciated that such physiological conditions resulting in bond breaking do not necessarily include a biological or metabolic process, and instead may include a standard chemical reaction, such as a hydrolysis reaction, for example, at physiological pH, or as a result of compartmentalization into a cellular organelle such as an endosome and/or lysosome having a lower pH than cytosolic pH.

Further preferably, L is a bond.

Moreover preferably, L is an optionally substituted heteroalkyl group containing from 1 to 20 carbon atoms and from 1 to 12 heteroatoms selected from O, S and N and wherein the preferred substituent is NH₂.

Further preferably, L comprises a -S-S- group or forms an -S-S- group with Pep or A.

Morover preferably, L comprises a group of formula -S-CH₂-CH₂-O-C(=O)-, -S-CH₂-CH(NH₂)-C(=O)- and/or -N-CH(CO₂H)-CH₂-S- and/or a tripeptide, tetrapeptide, pentapeptide, or hexapeptide preferably consisting of amino acids selected from the group consisting of aspartic acid, cysteine, glutamic acid, lysine, arginine, and ornithine, and combinations thereof.

Moreover preferably L is a linker described in US 2010/0004276, EP 2 481 427, WO 2006/012527 and WO 2008/112873, all of which are incorporated herein by reference. Especially preferably, L is selected from the linkers described in EP 2 481 427 A1 in paragraphs [0039] to [0132] and in claims 6 to 12.

Especially preferably, L is selected from the following groups:
a bond

   -CH₂-CH₂-S-

   -O-CH₂-CH₂-S-;

   -NH-CH₂-CH₂-S-; or

   -NH-NH-C(=O)-O-CH₂-CH₂-S-.

Preferably, therein the left side of L is bound to A and the right side is bound to Pep.

Further especially preferably, L is selected from the following groups:

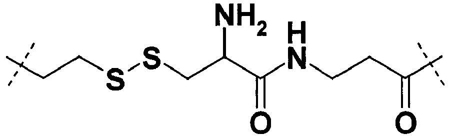

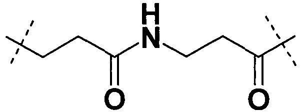

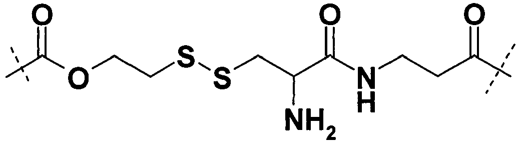

Preferably, therein the left side of L is bound to A and the right side is bound to Pep.

Preferably, Pep is a group (peptide residue) selected from:
H-Tyr-Pro-Ser-Lys(β-Ala-Cys-X^{L})-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂;
H-Tyr-Pro-Ser-Lys(β-Ala-p-Ala-X^{L})-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂; H-Tyr-Pro-Ser-Lys(β-Ala-X^{L})-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂;
H-Tyr-Pro-Ser-Lys(X^{L})-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂;
H-Tyr-Pro-Ser-Lys-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH-X^{L}; and
X^{L}-Tyr-Pro-Ser-Lys-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂,
wherein X^{L} denotes the point of attachement to L.

Further preferably, Pep is the following peptide (PepX): H-Tyr-Pro-Ser-Lys-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂ (PepX, SEQ ID NO: 1), wherein one hydrogen atom is replaced by the bond to L.

The term alkyl or alk refers to a saturated, linear or branched, optionally substituted hydrocarbon group, containing preferably from one to twenty carbon atoms, preferably from one to twelve carbon atoms, mostly preferred from one to six carbon atoms, for example methyl, ethyl, propyl, isopropyl, isobutyl, n-butyl, sek-butyl, tert-butyl, n-pentyl, 2,2-dimethylpropyl, 2-methylbutyl, n-hexyl, 2,2-dimethylbutyl or 2,3-dimethylbutyl.

The term alkenyl and alkinyl refers to an at least partially unsaturated, linear or branched, optionally substituted hydrocarbon group, containing preferably from two to twenty carbon atoms, preferably from two to twelve carbon atoms, mostly preferred from two to six carbon atoms, for example ethenyl, allyl, acetylenyl, propargyl, isoprenyl, or hex-2-enyl. Preferentially, alkenyl groups contain one or two, most preferred one double bond and alkinyl groups contain one or two, most preferred one triple bond.

Optionally the terms alkyl, alkenyl and/or alkinyl refer to groups where one or several, preferentially one, two or three hydrogen atoms are replaced by a halogen atom, preferentially fluorine or chlorine or a 2,2,2-trichlorethyl, or a trifluoromethyl group.

The term heteroalkyl refers to an alkyl, alkenyl or alkinyl group, where one or more, preferentially one, two or three carbon atoms are replaced by an O, N, P, B, Se, Si, or S atom, preferentially O, S or N. The term heteroalkyl also refers to a carboxylic acid or a group derived thereof, for example acyl (alkyl-CO), acylalkyl, alkoxycarbonyl, acyloxy, acyloxyalkyl, carboxyalkylamid or alkoxycarbonyloxy.

Examples of heteroalkyl groups are groups of the formula R^{a}-O-Y^{a}-, R^{a}-S-Y^{a}-, R^{a}-N(R^{b}) -Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b}) -Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N (R^{b})-CS-N(R^{c})-Y^{a}-, R^{a}-O-CS-O-y^{a}-, R^{a}-S-CO-Y^{a}-, R^{a}-CO-S-Y^{a}-, R^{a}-S-CO-N(R^{b}) -Y^{a}-, R^{a}-N(R^{b}) -CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} refers to a H, a C₁-C₆-alkyl, a C₂-C₆-alkenyl or a C₂-C₆-alkinyl group; wherein R^{b} refers to a H, a C₁-C₆-alkyl, a C₂-C₆-alkenyl or a C₂-C₆-alkinyl group; wherein R^{c} refers to a H, a C₁-C₆-alkyl, a C₂-C₆-alkenyl or a C₂-C₆-alkinyl group; wherein R^{d} refers to a H, a C₁-C₆-alkyl, a C₂-C₆-alkenyl or a C₂-C₆-alkinyl group and Y^{a} refers to a direct binding, a C₁-C₆-alkylen, a C₂-C₆-alkenylen or a C₂-C₆-alkinylen group, wherein each heteroalkyl group can be replace by a carbon atom and one or several hydrogen atoms can be replaced by fluorine or chlorine atoms. Examples of heteroalkyl groups are methoxy, trifluormethoxy, ethoxy, n-propyloxy, iso-propyloxy, tert-butyloxy, methoxymethyl, ethoxymethyl, methoxyethyl, methylamino, ethylamino, dimethylamino, diethylamino, iso-propylethylamino, methyl-aminomethyl, ethylaminomethyl, di-iso-propylaminoethyl, enolether, dimethylaminomethyl, dimethylaminoethyl, acetyl, propionyl, butyryloxy, acetyloxy, methoxycarbonyl, ethoxy-carbonyl, N-ethyl-N-methylcarbamoyl or N-methylcarbamoyl. Other examples of heteroalkyl groups are nitrile, isonitrile, cyanate, thiocyanate, isocyanate, isothiocyanate and alkylnitrile groups.

The term cycloalkyl refers to a saturated or partially unsaturated (e.g. cycloalkenyl) optionally substituted cyclic group, comprising one or several rings, preferentially one or two rings, containing three to fourteen ring carbon atoms, preferentially three to ten, preferentially three, four, five, six or seven ring carbon atoms. Furthermore the term cycloalkyl refers to a group where one or more hydrogen atoms are replaced by F, Cl, Br, I, OH, =O, SH, =S, NH₂, =NH, or NO₂, or cyclic ketones, for example cyclohexanone, 2-cyclohexenone or cyclopentanone. Examples of cycloalkyl groups are cyclopropyl, cyclobutyl, cyclopentenyl, spiro[4,5]-decanyl, norbornyl, cyclohexyl, cyclopentenyl, cyclohexadienyl, decalinyl, cubanyl, bicyclo[4.3.0]nonyl, tetralin, cyclopentylcyclohexyl, fluorcyclohexyl or the cyclohex-2-enyl group.

The term heterocycloalkyl refers to a cycloalkyl as defined above, wherein one or several, preferentially one, two or three ring carbon atoms are replaced by an O, N, Si, Se, P, or S, preferentially O, S or N. Preferentially a heterocycloalkyl group is composed of one or two rings comprising three to ten, preferentially three, four, five, six or seven ring atoms. Moreover the term heterocycloalkyl refers to groups where one or several hydrogen atoms are replaced by F, Cl, Br, I, OH, =O, SH, =S, NH₂ or NO₂. Examples of heterocycloalkyl are piperidyl, morpholinyl, urotropinyl, pyrrolidinyl, tetrahydrothiophenyl, tetrahydropyranyl, tetrahydro-furyl, oxacyclopropyl, azacyclopropyl or 2-pyrazolinyl groups as well as lactams, lactons, cyclic imides and cyclic anhydrides.

The term alkylcycloalkyl refers to groups, which contain cycloalkyl as well as alkyl, alkenyl or alkinyl groups according to the above definition, e.g. alkylcycloalkyl, alkylcycloalkenyl, alkenylcycloalkyl and alkinylcycloalkyl groups. Preferentially an alkylcycloalkyl group is composed of a cycloalkyl group, comprising one or more rings, comprising three to ten, preferentially three, four, five, six or seven carbon atoms and one or two alkyl, alkenyl oder alkinyl groups with one or two to six carbon atoms.

The term heteroalkylcycloalkyl refers to alkylcycloalkyl groups, according to the above definition, wherein one or several, preferentially one, two or three carbon atoms are replaced by O, N, Si, Se, P or S, preferentialy O, S or N. Preferentially it is composed of one or two ring systems with three to ten, preferentially three, four, five, six or seven ring atoms and one or two alkyl, alkenyl, alkinyl or heteroalkyl groups with one or two to six carbon atoms. Examples of such a group are alkylheterocycloalkyl, alkylheterocycloalkenyl, alkenylheterocycloalkyl, alkinylheterocycloalkyl, heteroalkylcycloalkyl, heteroalkylheterocycloalkyl and heteroalkylheterocylcloalkenyl, wherein the cyclic group is saturated or partially (simply, twofold or threefold) unsaturated.

The term aryl or ar refers to an optionally substituted aromatic group, composed of one or several rings, comprising six to fourteen carbon atoms, preferentially six to ten, preferentially six carbon atoms. The term aryl or ar can also refer to an aromatic group, wherein one or several H atoms are replaced by F, Cl, Br or I or OH, SH, NH2, or NO2. Examples are phenyl-, naphthyl-, biphenyl-, 2-fluorphenyl, anilinyl-, 3-nitrophenyl or 4-hydroxy-phenyl.

The term heteroaryl refers to an aromatic group, composed of one or several rings, comprising five to fourteen ring atoms, preferentially five to ten, whereof one or several, preferentially one, two, three or four are O, N, P or S ring atoms, preferentially O, S or N. The term heteroaryl can also refer to groups, wherein one or several H atoms are replaced by F, Cl, Br or I or OH, SH, NH2, or NO2. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, chinolinyl, purinyl, carbazolyl, acridinyl, pyrimidyl, 2,3'-bifuryl, 3-pyrazolyl and isochinolinyl.

The term aralkyl (or arylalkyl or alkylaryl) refers to groups composed of aryl and alkyl, alkenyl, alkinyl and/or cycloalkyl, e.g. arylalkyl, arylalkenyl, arylalkinyl, arylcycloalkyl, arylcycloalkenyl, alkylarylacycloalkyl and alkylarylcycloalkenyl. Examples of aralkyles are toluol, xylol, mesitylen, styren, benzylchloride, o-fluortoluene, 1H-inden, tetralin, dihydronaphthaline, indanon, phenylcyclopentyl, cumol, cyclo-hexylphenyl, fluoren and indan. Preferentially, an aralkyl group is composed of one or two aromatic rings, comprising six to ten ring carbon atoms and one or two alkyl, alkenyl and/or alkinyl comprising one or two to six carbon atoms and/or one cyclo-alkyl comprising five or six ring carbon atoms.

The term heteroaralkyl (or heteroarylalkyl or heteroalkylaryl) refers to an aralkyl group as defined above, wherein one or several, preferentially one, two, three or four carbon atoms are replaced by O, N, Si, Se, P, B or S, preferentially O, N or S, and to groups which contain aryl, heteroaryl and alkyl, alkenyl, alkinyl and/or heteroalkyl and/or cycloalkyl and/or heterocycloalkyl. Preferentially a heteroaralkyl group is composed of one or two aromatic ring systems comprising five or six to ten carbon atoms and one or two alkyl, alkenyl and/or alkinyl comprising one or two to six carbon atoms and/or one cycloalkyl comprising five or six ring carbon atoms, wherein one, two, three or four carbon atoms can be replaced by O, N or S.

Examples are arylheteroalkyl, arylheterocycloalkyl, aryl-heterocycloalkenyl, arylalkylheterocycloalkyl, aryl-alkenylheterocycloalkyl, arylalkinylheterocyclo-alkyl, arylalkylheterocycloalkenyl, heteroarylalkyl, heteroarylalkenyl, heteroarylalkinyl, heteroarylheteroalkyl, heteroarylcycloalkyl, heteroarylcycloalkenyl, heteroarylheterocycloalkyl, heteroarylheterocycloalken-yl, heteroarylalkylcycloalkyl, heteroarylalkylhetero-cycloalkenyl, heteroarylheteroalkylcycloalkyl, heteroarylheteroalkylcycloalkenyl and heteroarylheteroalkyl heterocycloalkyl, wherein the cyclic groups can be saturated or once, twice, three fold of four fold unsaturated. Examples are tetrahydroisochinolinyl, benzoyl, 2- or 3-ethyl-indolyl, 4-methylpyridino, 2-, 3- or 4-methoxyphenyl, 4-ethoxyphenyl, 2-, 3- or 4-carboxyphenylalkyl.

The terms cycloalkyl, heterocycloalkyl, alkylcyclo-alkyl, heteroalkylcycloalkyl, aryl, heteroaryl, aralkyl and heteroaralkyl also refer to groups, wherein one or several H atoms are replaced by F, Cl, Br or I or OH, SH, NH₂, NO₂.

The term "optionally substituted" relates to groups, wherein one or several H atoms can be replaced by F, Cl, Br or I or OH, SH, NH₂, or NO₂. This term relates further to groups, which can be exclusively or additionally substituted with unsubstituted C₁-C₆ alkyl, C₂-C₆ alkenyl, C₂-C₆ alkinyl, C₁-C₆ heteroalkyl, C₃-C₁₀ cycloalkyl, C₂-C₉ heterocycloalkyl, C₆-C₁₀ aryl, C₁-C₉ heteroaryl, C₇-C₁₂ aralkyl or C₂-C₁₁ heteroaralkyl groups.

Protecting groups are known to a person skilled in the art and described in P. J. Kocienski, Protecting Groups, Georg Thieme Verlag, Stuttgart, 1994 and in T. W. Greene, P. G. M. Wuts, Protective Groups in Organic Synthesis, John Wiley & Sons, New York, 1999. Common amino protecting groups are e.g. t-butyloxycarbonyl (Boc), benzyloxycarbonyl (Cbz, Z), benzyl (Bn), benzoyl (Bz), fluorenylmethyloxycarbonyl (Fmoc), allyloxycarbonyl (Alloc), trichlorethyloxycarbonyl (Troc), acetyl or trifluoracetyl.

The compounds described herein can comprise several chiral centers depending on their substitution pattern. The present invention relates to all defined enantio- and diastereo isomers as well as their mixtures in all ratios. Moreover, the present invention relates to all cis/trans isomers of the compounds described herein as well as their mixtures. Moreover, the present invention relates to all tautomeric forms of the compounds described herein.

Examples of pharmacologically acceptable salts of the compounds described herein are physiologically acceptable mineral acids, e.g. hydrochloric acid, sulfuric acid, phosphoric acid or salts of organic acids, e.g. methansulfonic acid, p-toluenesulfonic acid, lactic acid, formic acid, trifluoracetic acid, citric acid, succinic acid, fumaric acid, maleic acid and salicylic acid. The compounds described herein can be solvated, especially hydrated. The hydration can occur during the synthesis process or can be a consequence of the hygroscopic nature of the originally dehydrated compounds described herein. As mentioned above, compounds described herein, containing asymmetric carbon atoms might exist as mixtures of diastereomers, as mixtures of enantiomers or as optically pure compounds.

The pharmaceutical compositions according to the present invention are composed of at least one compound described herein and optionally carriers and/or adjuvants.

Prodrugs are also subject of the present invention and they are composed of a compound described herein and at least one pharmacologically acceptable protecting group, which is cleaved under physiological conditions, e.g. alkoxy, aralkyloxy, acyl or acyloxy, more precisely ethoxy, benzyloxy, acetyl or acetyloxy.

The therapeutic use of the compounds described herein, their pharmacologic acceptable salts and/or solvates and hydrates, as well as the corresponding formulations and pharmacological compositions are also subject of the present invention.

The use of the compounds described herein for the preparation of drugs for the treatment of cancer is also subject of the present invention. Moreover, the present compounds are of interest for the prevention and/or treatment of rheumatoid arthritis, inflammatory diseases, immunological diseases (e.g. type I diabetis), autoimmune diseases, diseases of the eye, e.g. AMD (age related macular disease) or diabetic retinopathy other tumor diseases as well as for the surface treatment (impregnation) of plastic and metal implants, e.g. stents.

Especially, the compounds described herein are of interest for the treatment of cancers such as cancer types in which the NPY-1 receptor is overexpressed, such as breast cancer and especially metastatic breast cancer, and also in other cancer conditions such as renal cell carcinomas, gastrointestinal stromal tumors, nephroblastomas, neuroblastic tumors, paragangliomas, pheochromocytomas, adrenal cortical tumors, ovarian sex cord-stromal tumors, and ovarian adeno carcinomas.

In general, the compounds described herein can be given as a single treatment or as multiple treatments either alone or in combination with an arbitrary therapeutic substance according to known and accepted modes or as a continuous treatment whereby the active principle can be embedded in a matrix such as e.g. an implantable hydrogel.

Compositions according to the invention can be administered in one of the following ways: solutions, emulsions or suspensions; parenteral, including injectable solutions; by inhalation, including powder formulation or as a spray, transdermal or intranasal. For the production of liquid solutions and syrups one may use carriers for example water, alcohols, aqueous saline, aqueous dextrose, polyole, glycerin, vegatable oils, petroleum, animal or synthetic oils. For the production of suppositories one may use excipients like e.g. vegetable, petroleum, animal or synthetic oils, wax, fat and polyols. For aerosol formulations one may use compressed gases suitable for this purpose like e.g. oxygen, nitrogen, noble gas and carbon dioxide. The pharmaceutically useful agents may also contain additives for conservation, stabilisation, e.g. UV stabilizer, emulsifier, sweetener, aromatiser, salts to change the osmotic pressure, buffers, coating additives and antioxidants.

Combinations with other therapeutic agents can include further agents, which are commonly used to treat the diseases mentioned above, especially cancer.

Compounds of Formula (I), (II) and (III) can e.g. produced from building blocks that can be linked via peptide coupling methods using known coupling reagents, e.g. hydroxybenzotriazole (HOBt) and diisopropylcarbodiimide (DIC) or dicyclohexylcarbodiimide (DCC). Unless otherwise defined, all residues are defined as herein above.

Tubulysines and derivatives thereof are known to a person skilled in the art and can e.g. be prepared as described in WO 2008/138561, WO 2004046170, WO 2004/005327, WO 2011/057806, WO 2011/057805 and documents cited therein.

Epothilones and derivatives thereof are known to a person skilled in the art and can e.g. be prepared as described in Nicolaou et al. Angew. Chem. Int. Ed. 1998, 37, 2014-2045, WO 2004/007492, WO 2004/048372 and documents cited therein.

### Examples

The following derivatives were synthesized from building blocks A, L and Pep. The building blocks were synthesized according to methods known to a person skilled in the art.

### Automated Solid Phase Peptide Synthesis of Pep

Peptides were synthesized according to the Fmoc/tBu strategy using an automated multiple solid-phase peptide synthesizer Syro II (MultiSynTech GmbH, Bochum, Germany). To gain C-terminal peptide amides, a Rink amide resin with a loading capacity of 0.63 mmol/g was used.

Prior to each amino acid coupling step the base-labile N^{α}-protecting group Fmoc had to be cleaved off from the building blocks, and in a first step from the Rink amide resin as well. For Fmoc cleavage, 400 µL piperidine in DMF (40% v/v) was added to the resin and incubated for 3 min while stirring. The deprotection was repeated with 400 µL piperidine in DMF (20% v/v) for 10 min. Subsequently, the resin was washed with 4x 600 µL DMF.

Amino acids were coupled by preincubation of the resin with 200 µL amino acid building block solution (0.5 M in DMF) and 100 µL 3 M Oxyma in DMF for 2 min. Subsequently, 100 µL 3.3 M DIC in DMF were added and the reaction was allowed to proceed for 40 min with stirring. After a washing step with 800 µL DMF, the coupling step was repeated once for each amino acid.

For the selective deprotection of a Dde-protected lysine residue, the fully protected, resin-bound peptide was incubated 12 x 10 min with 1 mL freshly prepared 3 % hydrazine in DMF. After each of the 12 steps, the resin was washed with DMF. After the first and the twelfth step, the removed hydrazine solution was collected, and its absorption was measured at 301 nm against a reference of fresh hydrazine in DMF. The Dde deprotection was successful if the absorption of the tenth fraction was < 0.1.

### Analytical and Preparative Cleavage from Resin

For analytical purposes, a small amount of peptide-loaded resin was incubated with TFA/thioanisole/1,2-ethanedithiol (900:70:30 v/v) for 3 h at room temperature, removing all acid-labile protecting groups. Subsequently, the peptide was precipitated for 20 min at -20 °C in 1 mL ice cold diethyl ether, collected by centrifugation (2 min at 7,000 g), and washed with ice cold diethyl ether at least five times. The peptide pellet was dried and finally dissolved in 100 µL H₂O/tBuOH (1:3 v/v) for analysis.

For preparative cleavage, the complete resin was treated as described. However, precipitation occurred in 10 mL ice cold diethyl ether and centrifugation was performed at 4,400 g. The peptide was dried by using a SpeedVac, and finally lyophilized from 1 - 2 mL H₂O/tBuOH (1:3 v/v).

### Analytical RP-HPLC

To analyse the synthesized peptides by analytical RP-HPLC on a reversed phase Phenomenex Jupiter Proteo C18 column (4.6 mm x 250 mm, 5 µm), and an elution system composed of (A) 0.1% TFA in H₂O and (B) 0.08% TFA in ACN was used. A linear gradient of 20-70% solvent B in A over 40 min with a flow rate of 0.6 mL min-1 was used. The peptides were detected at 220 nm.

### Preparative RP-HPLC

Purification of the synthesized peptides was achieved by preparative RP-HPLC on a Phenomenex Jupiter Proteo C18 column (21.2 mm x 250 mm) using an elution system composed of (A) 0.1% TFA in H₂O and (B) 0.08% TFA in ACN, and an appropriate linear gradient of solvent B in A over 40-50 min and a flow rate of 10 mL min-1. For peptide detection, absorption at 220 nm was measured. Fractions were manually taken and analysed by MALDI-TOF mass spectrometry and analytical RP-HPLC. As pure identified fractions were combined and lyophilized.

### MALDI-TOF Mass Spectrometry

For mass analysis using MALDI-TOF mass spectrometry, a matrix consisting of 2,5-dihydroxybenzoic acid and 2-hydroxy-5-methoxybenzoic acid (10 g/L in ACN/H₂O/TFA 50 : 49.7 : 0.3 v/v) was used. A Bruker Daltonis Ultraflex III TOF/TOF was used for MALDI measurements.

### ESI Ion Trap Mass Spectrometry

For mass analysis using ESI Ion Trap mass spectrometry, the samples were diluted to 20 µM in H₂O (0.1% HCOOH) with ACN (7:3 v/v), injected and analysed. A Bruker HCT mass spectrometer was used for ESI measurements.

### Coupling of building block A-L to Pep via disulfide linkage

To couple OC169, OC503, OC504, OC505, OC506 via a disulfide linkage to K4(βAla-Cys)-[F7,P34]-NPY, the purified peptide was dissolved in 0.1 mM phosphate buffer according to Sörensen (pH 6.0) and degased using argon. The coupling reaction was performed under equimolar conditions (A-L and peptide) at room temperature. After 60 min the reaction was complete and product identity was confirmed by MALDI-TOF mass spectrometry. The product was purified immediately by preparative RP-HPLC.

### Coupling of building block A-L to Pep via peptide linkage

To couple OC501 or OC503 via a peptide bond to K4(βAla-βAla)Cys)-[F7,P34]-NPY or [K4,F7,P34]-NPY, standard amide coupling was used as described above after the described selective deprotection of the lysine residue. The coupling reaction was performed under equimolar conditions (A-L and peptide) at room temperature. Subsequently, the final product was cleaved from the resin. Product identity was confirmed by MALDI-TOF mass spectrometry. The product was purified immediately by preparative RP-HPLC.

### Signal Transduction

Cos-7 cells, stably transfected with the cDNA encoding the human Y1 receptor C-terminally fused to EYFP and the human Y2 receptor C-terminally fused to EYFP as well as the chimeric G protein Gα_{Δ6qi4myr} (Cos7_hY1R_EYFP_Gα_{Δ6qi4myr} and Cos7_hY2R_EYFP_Gα_{Δ6qi4myr}), were seeded into 24 well-plates. 24 hours after seeding, cells were incubated for 16 hours with ³H-myo-inositol solution (300 µl DMEM/ 0.6 µl ³H-myo-inositol per well). Subsequently, the cell culture medium was removed and cells were washed with 500 µl DMEM containing 10 mM LiCl. After 1 hour stimulation with different peptide concentrations (10⁻⁵ to 10⁻¹² M) in DMEM containing 10 mM LiCl was performed. ³H-inositol phosphates were accumulated. After stimulation, the samples were hydrolysed with 150 µl 0.1 N NaOH for 5 minutes. Neutralization was carried out by addition of 50 µl 0.2 M formic acid. The samples were subsequently diluted in IP dilution buffer and the cell pellet was removed with a pipette. ³H-inositol phosphates were isolated by anion exchange chromatography.

### Internalization Studies

HEK293 cells stably transfected with the human Y1 receptor C-terminally fused to EYFP (HEK293_hY1_EYFP) and the human Y2 receptor C-terminally fused to EYFP and an HA tag (HEK293_HA_hY2_EYFP) were seeded into sterile µ-slide 8 well-plates (ibidi GmbH, Martinsried, Germany) and cultivated until 80% confluency was reached. Cells were cultivated for 30 minutes in OptiMEM before ligand stimulation. Cell nuclei were stained with HOECHST 33342 nuclear dye. Cells were stimulated for 60 minutes with 1 µM NPY in OptiMEM at 37°C. Live cell images were obtained with an Axio Observer microscope and ApoTome imaging system (Zeiss, Jena, Germany). The fluorescence images were analyzed with the Zeiss Axio Viosion software Release 3.0.

### Destabilization of Tubulin Network

HEK293 cells stably transfected with the human Y1 receptor C-terminally fused to EYFP (HEK293_hY1_EYFP) and the human Y2 receptor C-terminally fused to EYFP and an HA tag (HEK293_HA_hY2_EYFP) were seeded into sterile µ-slide 8 well-plates (ibidi GmbH, Martinsried, Germany) and cultivated at 37°C. Cells were transfected with pTub_ECFP_C1 (1 µg DNA per well, 1 µl Lipofectamine 2000 per well, transfection for 60 min at 37 °C). Expression of Tubulin_ECFP was allowed over night. The next day, cells were stimulated with OC500 or free cytolysin for 16h in OptiMem. Live cell images were obtained with an Axio Observer microscope and ApoTome imaging system (Zeiss, Jena, Germany). The fluorescence images were analyzed with the Zeiss Axio Viosion software Release 3.0.

### Stability Studies in Buffer

K4(βAla-Cys-OC169)-F7,P34-NPY was diluted to 10⁻⁴ M in 0.1 M citrate buffer, pH 4 and 6, as well as in 0.2 M sodium acetate buffer, pH 4. Peptide identity was determined after 0h, 1h, 2h, 4h, 6h, 24h, 72h and 96h by ESI mass spectrometry.

### Stability studies in MCF-7 cell culture supernatant

K4(βAla-Cys-OC169)-F7, P34-NPY was diluted to 10⁻⁴ M in MCF-7 cell culture supernatant and peptide identity was determined after 0h, 1h, 3h, 5h, 7h and 24h by ESI mass spectrometry.

### Stability studies in reducing environment

K4(βAla-Cys-OC169)-F7,P34-NPY was diluted to 10⁻⁴ M in 20x cysteine in 0.1 M phosphate buffer, pH 6. Peptide identity was determined after 1 h and 24 h by ESI mass spectrometry.

### Cell proliferation assays

To evaluate the anti-proliferative and cytotoxic effect, respectively, of the compounds, a fluorometric resazurin-based cell viability assay was used. Human cancer and non-cancer cell lines (primarily breast cancer) were seeded with low densities into 96-well plates (1,500 - 6,000 cells per well), and were allowed to adhere for 24 h. Subsequently, the compounds - dissolved to appropriate concentrations in medium - were added to the cells. After 72 h under standard growth conditions, the incubation solution was replaced by 50 µM resazurin in medium, and the cells were incubated for 2 h. Finally, the conversion of resazurin to resorufin by viable, metabolically active cells was measured using a Synergy 2 multiwell plate reader (BioTek) with 540 nm excitation and 590 nm emission filter setting.

Alternative to 72 h compound incubations, pulsed experiments were conducted. In these cases, cells were incubated with compounds for e.g. 4, 6, 12, 18, 24, 36 or 48 h, then the incubation solution was replaced, the cells were rinsed once with cell culture medium, and subsequently were allowed to proliferate in compound-free medium until 72 h were reached. Resazurin reaction and measurement were done as written above.

### Apoptosis assays

Compound-mediated induction of apoptosis was examined using comercially available luminescent caspase assays (Promega). Human cancer and non-cancer cell lines (primarily breast cancer) were seeded with densities depending on the incubation time into 96-well plates (5,000 - 20,000 cells per well for 48 h incubations; 15,000 - 40,000 cells per well for 16 - 24 h incubations), and were allowed to adhere for 24 h. Subsequently, the compounds - dissolved to appropriate concentrations in medium - were added to the cells in 70 µL per well. Cells were incubated for 16 - 48 h under standard growth conditions. For the last hour of incubation, 10 µL/well of 8X resazurin solution (400 µM) were added in order to multiplex the caspase activities with the cell number/cell viability. When the incubation was finished, conversion of resazurin to resorufin was measured as described above. After that, Caspase-Glo reagent was added according to Promega's guidelines and incubated for 1 h at room temperature. Luminescence read-out was done using a Synergy 2 multiwell plate reader (BioTek).

Since fragmentation of the nuclei indicates ongoing apoptotic events as well, we examined whether CytoPep conjugates were able to induce nuclear fragmentation. For this purpose, HEK293 cells stably transfected with the human Y1 receptor C-terminally fused to EYFP (HEK293_hY1_EYFP) and the human Y2 receptor C-terminally fused to EYFP and an HA tag (HEK293_HA_hY2_EYFP) were seeded into sterile µ-slide 8 well-plates (ibidi GmbH, Martinsried, Germany) and cultivated to 80% confluency. Cells were stimulated with OC500 or free cytolysin for 16h in OptiMem. Nuclei were stained using HOECHST 33342 nuclear dye. Live cell images were obtained with an Axio Observer microscope and ApoTome imaging system (Zeiss, Jena, Germany). The fluorescence images were analyzed with the Zeiss Axio Viosion software Release 3.0.

### Y receptor knockdown using siRNA

To examine the biological effects of the compounds in dependence of the hY1 receptor density on the cell surface, siRNA was used for transient NPY1R knockdown. For specific targeting a siGENOME SMARTpool human NPY1R and as control a siGENOME non-targeting siRNA pool #2 (Dharmacon) were used.

Human Y1 receptor expressing breast cancer cell lines were seeded in 25 cm² culture flasks to reach - 50% confluency for cell transfection after - 24 h. Transient, liposomal cell transfection was performed using Metafectene Pro (Biontex). Briefly, per 25 cm² culture flask 0 - 400 pmol siRNA were preincubated in 500 µL PBS for 5 min at room temperature and, subsequently, gently mixed with 32 µL Metafectene Pro in 500 µL PBS. After 20 min complexation at room temperature the transfection mixture was added to 4 mL fresh culture medium (with 4 - 5% FCS, instead of 10%) per culture flask.

Incubation was stopped after 24 h, and the transfected cells were used as indicated. For proliferation or apoptosis studies cells were seeded into 96-well plates. To evaluate the receptor expression levels, cells were harvested and RNA extraction as well as RT-PCR was conducted.

### Expression analyses using RT-PCR

Samples for expression analyses were prepared by RNA extraction using the Bio&Sell RNA Mini Kit, followed by a DNase I digestion step and cDNA synthesis using RevertAid Premium Reverse Transcriptase (Fermentas). All methods are done according to the manufacturer's guidelines. Finally, expression of NPY Y receptors, estrogen and progesterone receptors etc. were analyzed by using appropriate primers and conventional PCR.

### Stable isotope labelling by amino acids in cell culture (SILAC)

SILAC was used for the examination of compound-mediated protein deregulations in the cells. In a first step, the incorporation rate of ¹³C-labeled L-arginine ("heavy" medium) was determined for the used cell lines.

When the incorporation rate was ≥ 95%, the compound incubation (e.g. for 24 h) startet with "light" medium for the untreated reference and "heavy" medium for treated cells. After finished incubation, the cells were harvested and lysed. Protein contents were quantified, 20 µg "light"-labeled protein extract was mixed with 20 µg "heavy"-labeled protein extract and separated on a 12% SDS-PAGE. The gel was cut into gel slices and in-gel digested with trypsin. The samples were measured with a LTQ Orbitrap Velos (Thermo Scientific) coupled with a nano-HPLC. The resulting mass spec-data were analyzed using the MaxQuant software. The mormalized ratios "heavy"/"light" were used to identify significantly up- or downregulated proteins (upregulation > 1.5; downregulation < 0.666).

### Data Analysis

For data analysis GraphPad Prism 5.03 and LibreOffice Calc were used.

### Animal xenograft studies

For in vivo efficacy studies, a subcutaneous human breast carcinoma xenograft model (MDA-MB-468) in NMRI nude mice was used. Female NMRI nude mice were 7 weeks when the tumor cells were inoculated with 5x10⁶ MDA-MB-468 cells per mouse (200 µL of cell suspension in PBS with 2.5x10⁷ cells/mL injected in the right flank).

Each experimental group consisted of 5 animals (untreated reference group and OC compound treated group). Treatment started with tumor volumes - 100 mm³ (range 80 - 120 mm³). Animals were treated iv in the lateral tail vein with doses of 1 µmol/kg (in sterile PBS; application volume 5 mL/kg) 3x per week for 2 weeks. Following the final treatment, the animals were observed for further 3 weeks.

Investigated parameters were: survival, clinical signs and animal behaviour (daily), body weight and tumor volume (2x per week). Tumor growth was monitored by calliper measurement and tumor volume was calculated according to the formula W²xL/2 (L = length and W = perpendicular width of the tumor, L > W).

At the end of the study, a necropsy was done, primary tumor net weights were determined, and serum and tumor samples were taken.

Pep1 and Pep 2 were synthesized as described above. The tubulysin derivatives were prepared as described in the literature given above.
Pep1:
   H-Tyr-Pro-Ser-Lys(β-Ala-β-Ala)-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂ MS-TOF: 4396.1 [M+H]⁺
Pep2:
   H-Tyr-Pro-Ser-Lys(β-Ala-Cys-H)-Pro-Asp-Phe-Pro-Gly-Glu-Asp-Ala-Pro-Ala-Glu-Asp-Leu-Ala-Arg-Tyr-Tyr-Ser-Ala-Leu-Arg-His-Tyr-Ile-Asn-Leu-Ile-Thr-Arg-Pro-Arg-Tyr-NH₂ MS-ESI: 1108.3 [M+4H] ⁴⁺; MS-TOF: 4428.2 [M+H]⁺

OC-501: 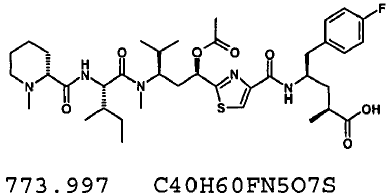 ESI analysis M⁺¹ 774.6 m/z (MW 773.4Da)

OC-169: 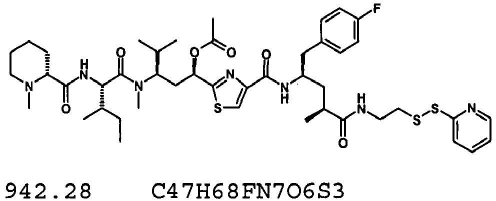 ESI analysis M⁺¹ 942.6 m/z (MW 941.4Da)

OC-506:

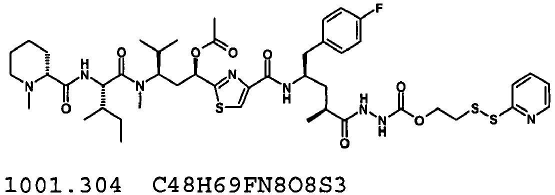

OC-503: 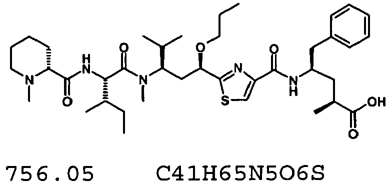 MS-ESI: 756.4 [M+H]⁺;

OC-504: 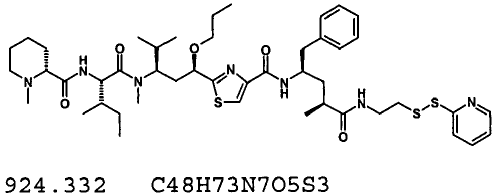 ESI analysis M⁺¹ 924.7 m/z (MW 923.5)

OC-505: 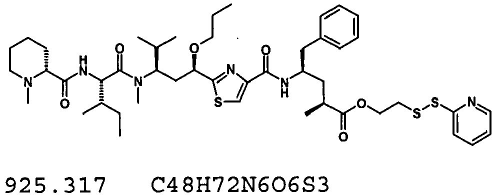 ESI analysis M⁺¹ 925.7 m/z (MW 924.5)

OC-509:

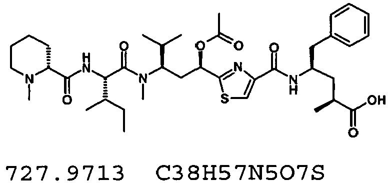

OC-175:
OC509 + Pep1 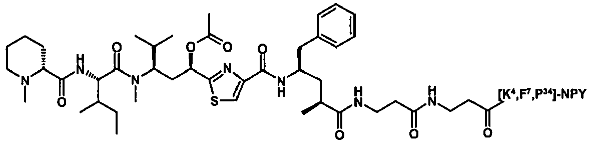 MS-ESI: 1028.0 [M+5H],⁵⁺;

OC 500:
OC-169 + Pep2 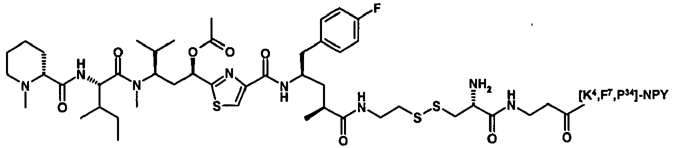 MS-ESI: 1053.0 [M+5H]⁵⁺; MS-TOF: 5258.5 [M+H]⁺

OC-508:
OC-506 + Pep2 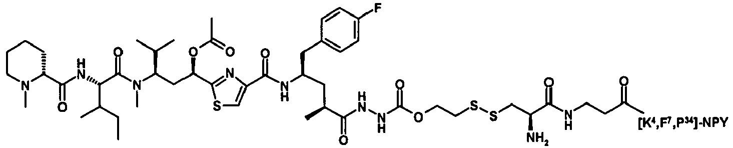 MS-TOF: 5317.5 [M+H]⁺

OC-510:
OC-504 + Pep2 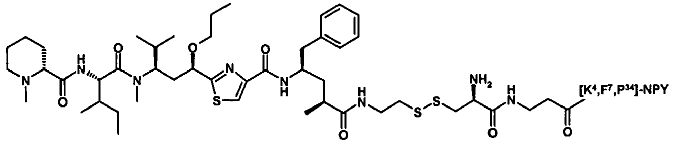 MS-TOF: 5242.8 [M+H]⁺;

**OC-511:**
OC-505 + Pep2 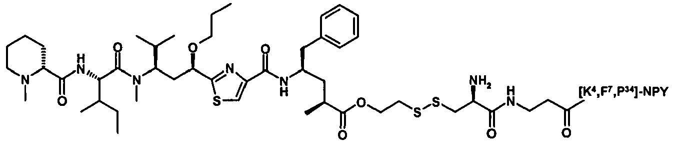 MS-ESI: 1049.6 [M+5H]⁵⁺ ;

Tubulysin A and Paclitaxel have been used as references. The results are shown in Table 1.

**Table 1: IC50 values determined in 72 h cell proliferation assays.**

| **Compound** | **cell lines - IC50 [nM]** | | | | | |
|---|---|---|---|---|---|---|
| | MCF-7 | T-47D | MDA-MB-231 | MDA-MB-468 | 184B5 | HT-29 |
| Tubulysin A (Reference) | 0.6 | | | | | 0.75 |
| Paclitaxel (Reference) | 25.5 | 30.7 | 35.1 | 31.1 | | |
| OC501 | 4.1 | 5.4 | 7.2 | 3.6 | | 1.2 |
| OC169 | 18.4 | 18.7 | 31.0 | 18.7 | 26.1 | |
| OC500 | 411 | 460 | 304 | 286 | 231 | |
| OC503 | 4.2 | 8.3 | 10.9 | 8.2 | | 0.8 |
| OC509 | 0.34 | | | | | |
| OC175 | 994 | | | | | |
| OC502 | > 1,000 | > 1,000 | > 1,000 | > 1,000 | | |

In general the new molecules according to this invention show an activity against several cancer cell lines between 0.03 to 60 nM.

### Signal Transduction

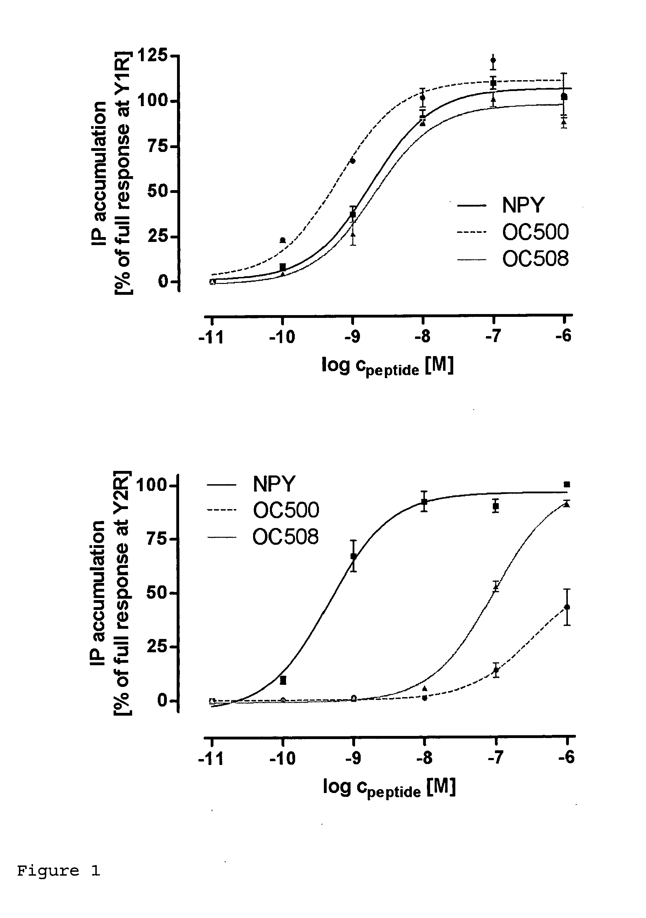

Figure 1 shows functional activation of human Y1 and Y2 receptors by NPY and the CytoPep conjugates OC500 and OC508 as determined by using an functional IP₃ assay.

**Table 2: EC₅₀s of Y receptor activation by NPY derivatives and CytoPep conjugates OC500 and OC508 as determined by using an functional IP₃ assay.**

| **Peptide** | **hY1R** | **hY2R** |
|---|---|---|
| | **EC50 [nM]** | **EC50 [nM]** |
| | **(pEC50 ± SEM)** | **(pEC50 ± SEM)** |
| OC500 | 0.6 | > 1,000 |
| | (9.2 ± 0.15) | |
| OC508 | 2.0 | 92.9 |
| | (8.7 ± 0.14) | (7.0 ± 0.05) |
| NPY | 1.7 | 0.5 |
| | (8.7 ± 0.07) | (9.3 ± 0.09) |
| [F⁷, P³⁴] -NPY | 1.4 | 135.6 |
| | (8.8 ± 0.10) | (6.7 ± 0.20) |
| hPP | n.d. | n.d. |

OC500 activates the human Y1 receptor with an sub-nanomolar EC₅₀, and is highly hY1 selective versus the human Y2 receptor. As determined, OC508 activates the human Y1 receptor with an low-nanomolar EC₅₀, i.e. around 50-times better compared with the human Y2 receptor.

### Internalization Studies

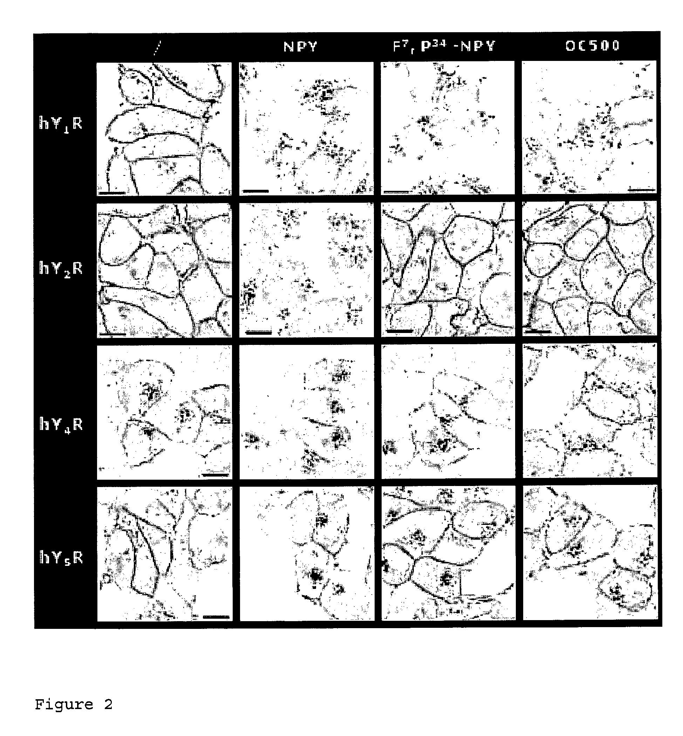

Fluorescence microscopic internalization studies revealed for [F⁷, P³⁴] -NPY and OC500 human Y1 receptor selective cellular internalization by endocytotic pathways (Figure 2). The endogeneous and unselective ligand NPY binds and internalizes with all of the four Y receptor species, indicated by the relocation of the EYFP-tagged receptors (shown in gark gray) from the plasma membrane to intracellular vesicles. With [F⁷,P³⁴]-NPY and OC500, this relocation - compared to an untreated reference - was observed just with cells expressing the hY1 receptor, and not with those expressing hY2, and to a minor extent with those expressing hY4 or hY5 receptors.

Figure 2 shows peptide-mediated cellular internalization of human Y receptor species. HEK293 cells stably expressing EYFP-tagged human Y1, Y2, Y4 and Y5 receptors (dark gray), respectively, were treated with 1 µM peptide for 1 h. Nuclei were stained with H33342 (light gray). Live cell images were taken with an Axio Observer microscope and ApoTome imaging system (Zeiss, Jena, Germany). Scale bars indicate 10 µm.

### Destabilization of Tubulin Network

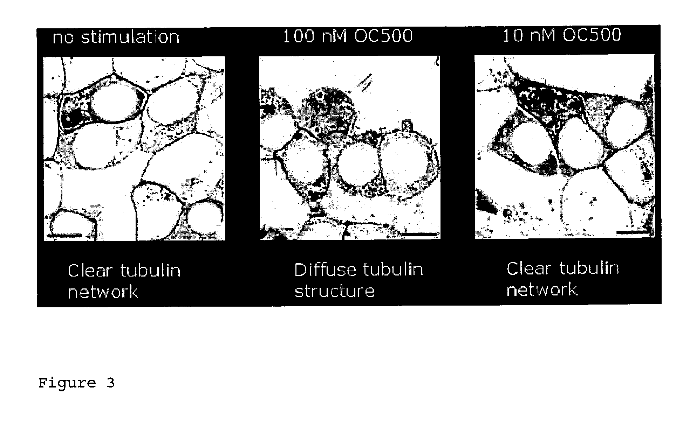

HEK293 cells, expressing ECFP-tagged Tubulin and EYFP-tagged human Y1 receptor, were used to investigate the effects of the compounds on the tubulin network integrity. For instance, treatment of the cells with 100 nM OC500 for 16 h caused a degraded tubulin network and a apoptosis-like cell morphology (Figure 3).

Figure 3 shows OC500-mediated destabilization of the cellular tubulin network. HEK293 cells, expressing EYFP-tagged human Y1 receptors (dark gray in membranes) and ECFP-tagged tubulin (mid gray in cell plasma) were treated with indicated OC500 concentrations for 16 h. Live cell images were taken with an Axio Observer microscope and ApoTome imaging system (Zeiss, Jena, Germany).

### Cell proliferation

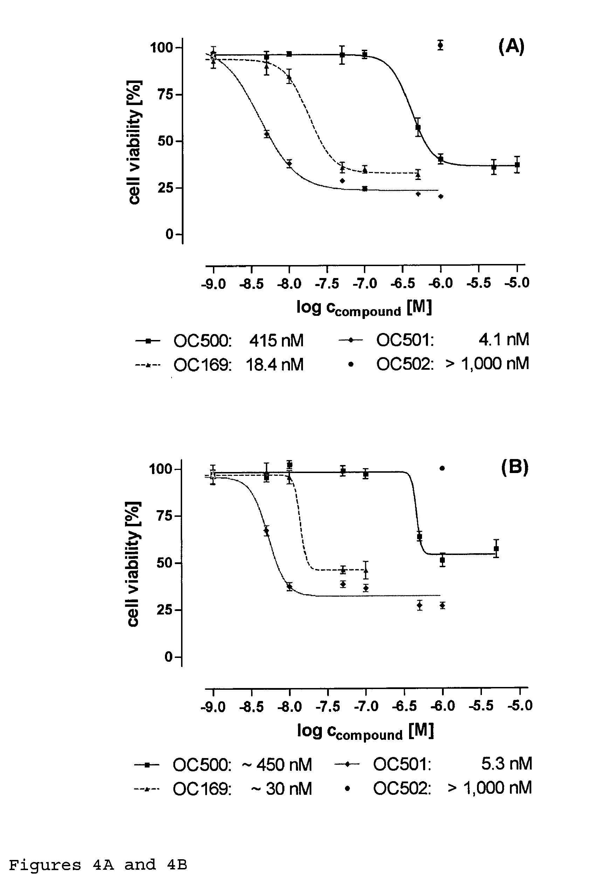

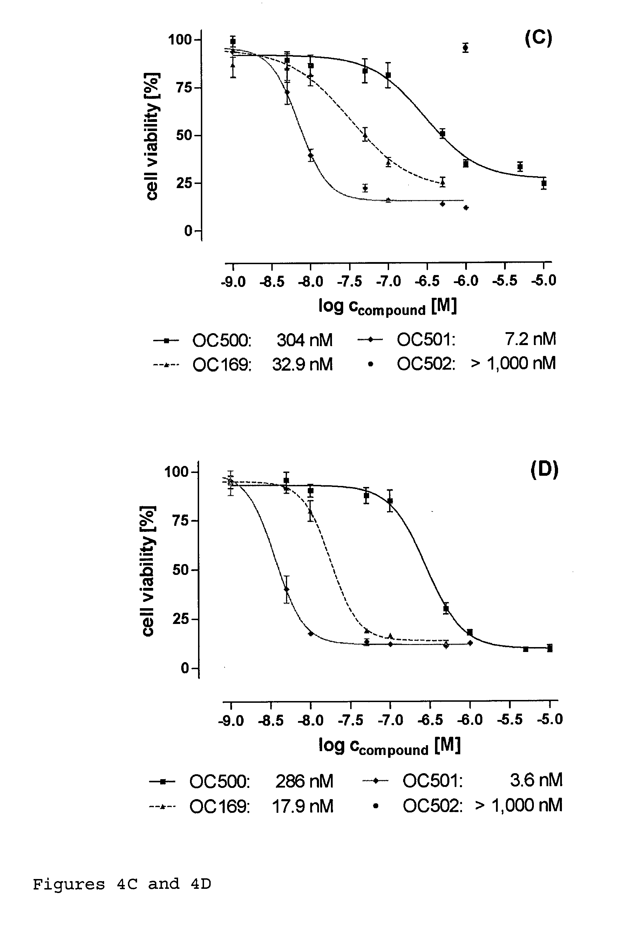

Cell proliferation assays with various breast cancer cell lines revealed that the free toxins, e. g. OC501, were - 5-fold more effective than the refernce compound paclitaxel. The linker-activated compounds (e. g. OC169) were active in the range of paclitaxel. The cytotoxic peptide conjugates (e. g. OC500) were active in the mid-nanomolar range, whereas the underlying peptide alone (e. g. OC502) showed no cytotoxicity. Generally, the peptide conjugates were more efficient against cells of the basal or triple negative breast cancer type (MDA-MB-231 and MDA-MB-468), whereas the hormone receptor positive cell lines MCF-7 and T-47D were more resistant (Figure 4).

Figures 4A-D show inhibition of cell proliferation of various breast cancer cell lines, (A) MCF-7, (B) T-47D, (C) MDA-MB-231, and (D) MDA-MB-468. Cells were treated with OC501 and its corresponding derivatives OC169 and OC500, as well as the underlying peptide OC502. Cell proliferation was detected after 72 h using a resazurin-based cell viability assay. The effects of the compounds are expressed as IC₅₀ values.

### Apoptosis assays

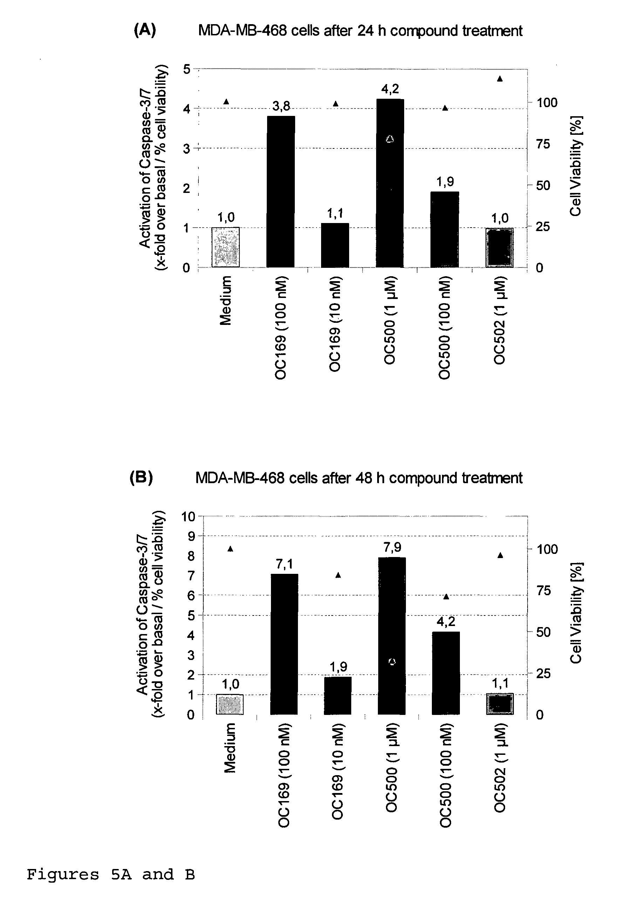

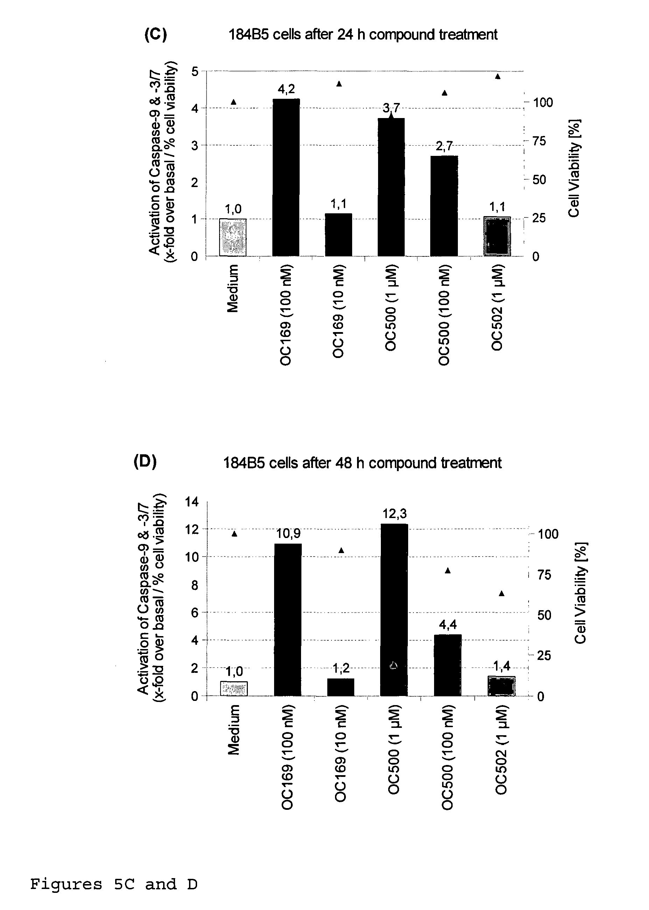

Investigations of activity levels of the effector caspases 3 and 7 in several breast cancer cell lines after compound treatment revealed the induction of apoptotic cell death mechanisms (caspase-dependent) by free cytolysins (e. g. OC169) as well as by the cytotoxic peptide conjugates (e.g. OC500), whereas the underlying peptide OC502 failed to trigger apoptotic events. As shown for MDA-MB-468 and 184B5 cells, the induction of apoptosis - and consequently reduced cell viability - was shown to be a time and concentration dependent effect (Figure 5).

Figures 5A-D show activation of caspases 3/7 (columns) and cell viability (triangle) of breast cancer cell lines MDA-MB-468 (A, B) and 184B5 (C, D). Cells were treated with the indicated compound concentrations for 24 h (A, C)and 48 h (B, D), respectively. Subsequently, activation of effector caspases 3/7 was measured using a luminescence-based caspase-Glo™ assay kit (Promega), and cell viability was fluorimetrically detected using a resazurin-based cell viability assay. Cell viability counts were used to normalize the caspase activity data, which are shown as x-fold over basal (untreated cells).

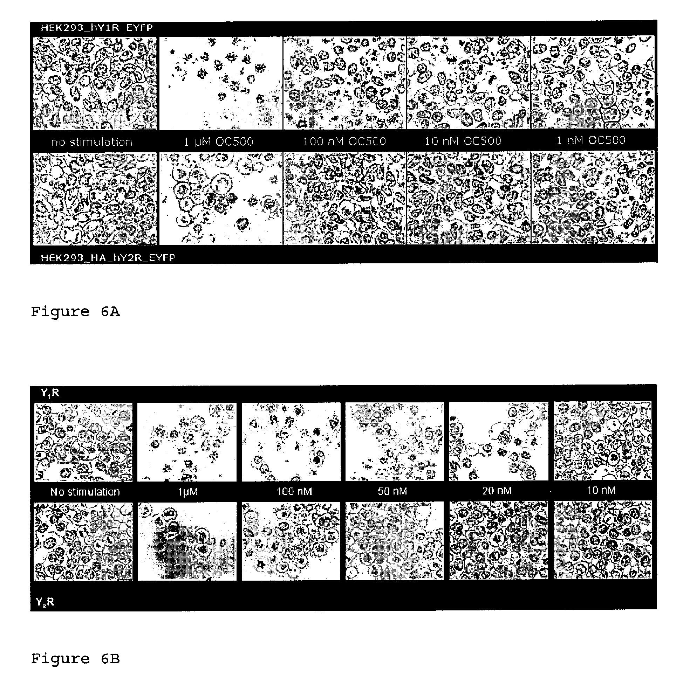

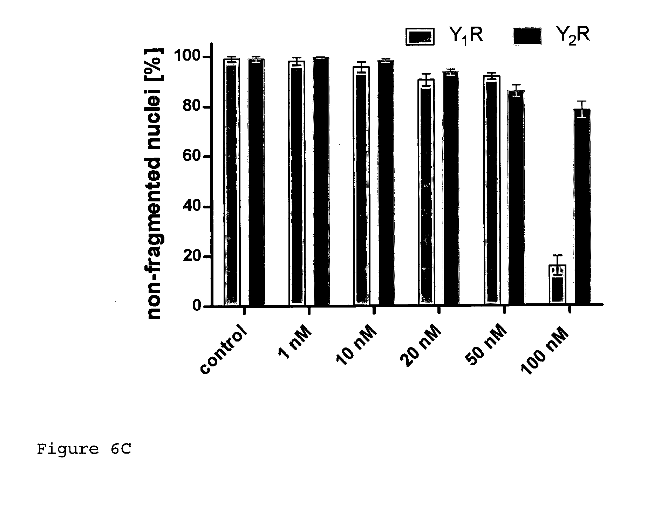

Nuclei fragmentation assays revealed for both compounds, OC500 and OC508, remarkable fragmentation of the nuclei of hY1 expressing cells at concentration of 50-100 nM and higher (Figure 6). In cells expressing hY2 receptors, the fragmentation was less at these compound concentrations. Therefore, OC500 as well as OC508 showed hY1 receptor selectivity, which was obviously more distinct with OC500. However, OC508 was slightly more effective. These findings are consistent with signal transduction data, and point to apoptotic mechanisms triggered by OC500 and OC508.

Figure 6A-C show nuclei fragmentation of HEK293 cells stably expressing the human Y1 and Y2 receptor, respectively. Cells were treated for 16 h with indicated concentrations of OC500 (A) and OC508 (B), respectively. Nuclei were stained using Hoechst 33342. Live cell images were taken with an Axio Observer microscope and ApoTome imaging system (Zeiss, Jena, Germany). Subsequently, the portion of fragmented nuclei were calculated.

### Stable isotope labelling by amino acids in cell culture (SILAC)

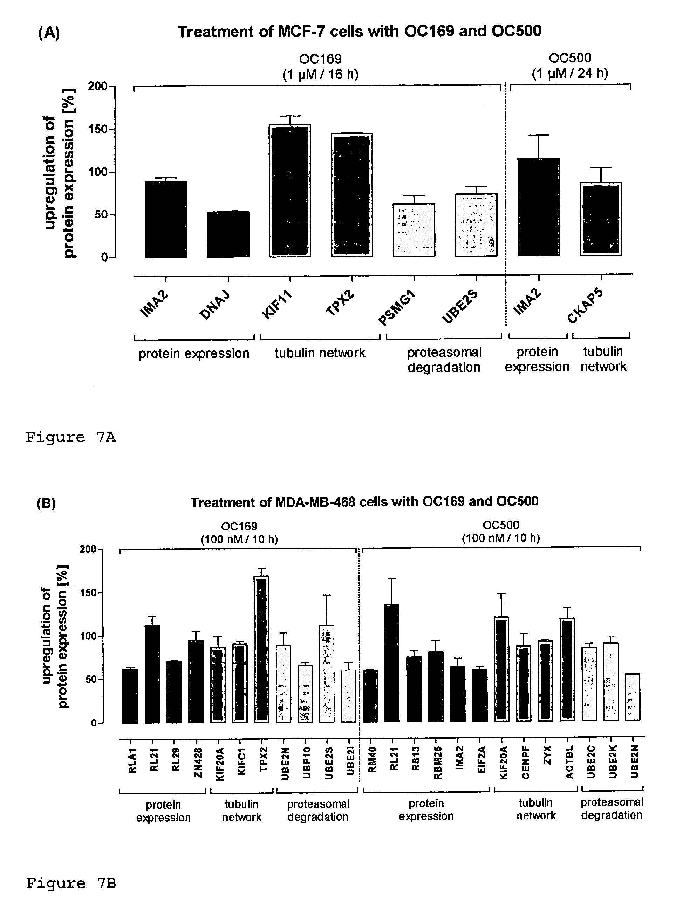

Proteomic analyses using SILAC were conducted to evaluate the effects of the compounds on the cellular regulation of protein expression (Figure 7). Treatment with e. g. OC169 and OC500, respectively, resulted in comparable protein regulations in various breast cancer cell lines. The observed upregulation of the expression of a number of proteins can be roughly classified into at least three functional groups, i. e. proteins dealing with the regulation of protein expression, cytoskeleton-related proteins, and those proteins dealing with proteolysis-associated events. In general, the regulation of all these proteins triggered by OC169 and OC500 can be seen in the context of the compounds induced degradation of the cellular tubulin network, and the effort of the cell to recycle and repair the cytoskeleton.

Figure 7 shows proteom analyses of MCF-7 (A) and MDA-MB-468 (B) cells after treatment with OC169 and OC500, respectively, using stable isotope labelling by amino acids in cell culture (SILAC). Cells were treated with indicated concentrations of the compounds for the indicated times, and up- or downregulated proteins were detected using nano-HPLC/mass spectrometry. Here, exclusively the upregulated proteins are shown, and classified into three functional groups, dealing with regulation of protein expression, organization of the tubulin network and proteasomal recycling processes. IMA2, importin subunit alpha-2; DNAJ, DnaJ homolog subfamily B member 1; KIF11, kinesin-like protein 11; TPX2, targeting protein for Xklp2; PSMG1, proteasome assembly chaperone 1; UBE2S, ubiquitin conjugating enzyme E2S; CKAP5, cytoskeleton-associated protein 5; RLA1, 60S acidic ribosomal protein P1; RL21, 60S ribosomal protein L21; RL29, 60S ribosomal protein L29; ZN428, zinc finger protein 428; KIF20A, kinesin-like protein 20A; KIFC1, kinesin-like protein C1; UBE2N, ubiquitin conjugating enzyme E2N; USP10, ubiquitin carboxy-terminal hydrolase 10; UBE2I, ubiquitin conjugating enzyme E2I; RM40, 39S ribosomal protein L40 (mitochondrial); RS13, 40S ribosomal protein S13; EIF2A, eukaryotic translation initiation factor 2A; CENPF, centromere protein F; ZYX, zyxin; ACTBL, beta-actin-like protein 2; UBE2C, ubiquitin conjugating enzyme E2C; UBE2K, ubiquitin conjugating enzyme E2K.

## Claims

1. A compound of Formula (I):
**A-L-Pep** (I)
wherein
Pep is selected from: 
H-Tyr-Pro-Ser-Lys(β-Ala-Cys-X^L)-Pro-Asp-Phe-Pro-Gly-Glu-Asp-
Ala-Pro-Ala-Glu-Asp-Leu-Ala-Arg-Tyr-Tyr-Ser-Ala-Leu-Arg-His-
Tyr-Ile-Asn-Leu-Ile-Thr-Arg-Pro-Arg-Tyr-NH₂;
 
H-Tyr-Pro-Ser-Lys(β-Ala-β-Ala-X^L)-Pro-Asp-Phe-Pro-Gly-Glu-Asp-
Ala-Pro-Ala-Glu-Asp-Leu-Ala-Arg-Tyr-Tyr-Ser-Ala-Leu-Arg-His-
Tyr-Ile-Asn-Leu-Ile-Thr-Arg-Pro-Arg-Tyr-NH₂;
 
H-Tyr-Pro-Ser-Lys(β-Ala-X^L)-Pro-Asp-Phe-Pro-Gly-Glu-Asp-Ala-
Pro-Ala-Glu-Asp-Leu-Ala-Arg-Tyr-Tyr-Ser-Ala-Leu-Arg-His-Tyr-
Ile-Asn-Leu-Ile-Thr-Arg-Pro-Arg-Tyr-NH₂;
 
H-Tyr-Pro-Ser-Lys(X^L)-Pro-Asp-Phe-Pro-Gly-Glu-Asp-Ala-Pro-Ala-
Glu-Asp-Leu-Ala-Arg-Tyr-Tyr-Ser-Ala-Leu-Arg-His-Tyr-Ile-Asn-
Leu-Ile-Thr-Arg-Pro-Arg-Tyr-NH2;
 
H-Tyr-Pro-Ser-Lys-Pro-Asp-Phe-Pro-Gly-Glu-Asp-Ala-Pro-Ala-Glu-
Asp-Leu-Ala-Arg-Tyr-Tyr-Ser-Ala-Leu-Arg-His-Tyr-Ile-Asn-Leu-
Ile-Thr-Arg-Pro-Arg-Tyr-NH-X^L;
 and 
X^L-Tyr-Pro-Ser-Lys-Pro-Asp-Phe-Pro-Gly-Glu-Asp-Ala-Pro-Ala-Glu-
Asp-Leu-Ala-Arg-Tyr-Tyr-Ser-Ala-Leu-Arg-His-Tyr-Ile-Asn-Leu-
Ile-Thr-Arg-Pro-Arg-Tyr-NH2,
 wherein X^{L} denotes the bond to L;
A is selected from tubulysins, natural and synthetic epothilones, auristatins, dolastatins, natural and synthetic vincristine, natural and synthetic vinblastine, amanitine, maytansines, taxanes, Nemorubicin, PNU-159682, pyrrolobenzodiazepins and dimers and duocarmycins wherein one hydrogen atom is replaced by the bond to linker L; and
L is a linker or a bond between A and Pep;
or a pharmacologically acceptable salt, a solvate, a hydrate or a pharmacologically acceptable formulation thereof.

2. The compound according to claim 1, wherein L is a bond or an optionally substituted alkylene, alkenylene, alkinylene, heteroalkylene, arylene, heteroarylene, cycloalkylene, heterocycloalkylene, alkylcycloalkylene, heteroalkylcycloalkylene, aralkylene or heteroaralkylene group.

3. The compound according to claims 1 or 2, wherein L is a bond or an optionally substituted heteroalkyl group containing from 1 to 20 carbon atoms and from 1 to 12 heteroatoms selected from O, S and N.

4. The compound of claim 1, 2 or 3, wherein A is a compound of Formula (Tub) 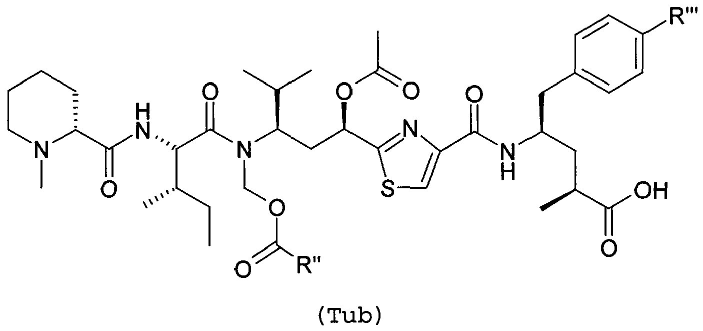 wherein R" is H, alkyl, alkenyl, aryl, or heteroaryl and R''' is H, OH, and wherein one hydrogen atom of the compound of Formula (Tub) is replaced by the bond to linker L;
or
wherein A is a compound of formula (IV), wherein one hydrogen atom is replaced by the bond to linker L: 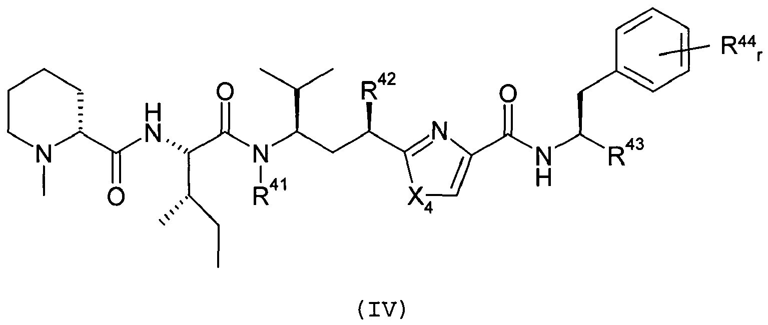 wherein
R⁴¹ is H or an alkyl, alkenyl, alkynyl, CO-alkyl, heteroalkyl, aralkyl or heteroaralkyl group, all of which may optionally be substituted;
R⁴² is OH, an alkyl, alkenyl, alkynyl, -O-alkyl, -O-alkenyl, - O-alkynyl, -O-CO-alkyl or heteroalkyl group, all of which may optionally be substituted;
R⁴³ is a group of formula CO₂H, CO₂R⁴⁵, CONHR⁴⁵, CONR₂⁴⁵, or a
group of formula CH₂OH or 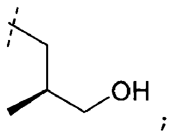 R⁴⁵ independently being an alkyl, aryl, aralkyl or heteroalkyl group;
X₄ is S or O;
R⁴⁴ is independently optionally substituted alkyl (e.g. methyl), optionally substituted heteroalkyl (e.g. OMe), halogen, CN, NO₂ or OH; and
r is 0, 1, 2, 3, 4 or 5.

5. The compound of claim 1, 2 or 3, wherein A is a compound of Formula (Epo) 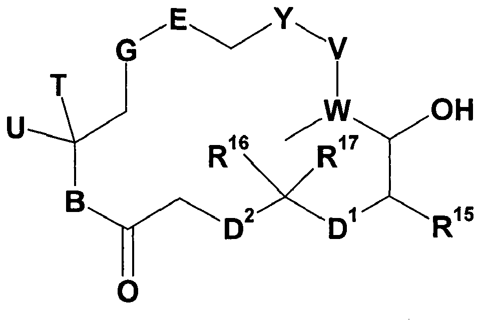 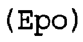 wherein
T is a heteroalkyl-, heterocycloalkyl-, heteroalkylcycloalkyl-, heteroaryl- or heteroarylalkyl-group,
U is hydrogen, halogen, an alkyl, heteroalkyl-, heterocycloalkyl-, heteroalkylcycloalkyl-, heteroaryl- or heteroarylalkyl-group,
G-E is selected from the following groups, 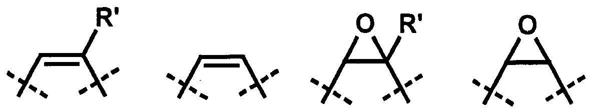 wherein R' is F or a C₁-C₃ alkyl group or G-E is part of an optionally substituted phenyl ring,
R¹⁵ is a C₁-C₄-alkyl-, a C₁-C₄-alkenyl-, a C₁-C₄-alkynyl- or a C₃-C₄-cycloalkyl-group,
Y-V-W is a group of formula CH=CH-CH, CH₂-CH₂-CH or CH₂-CH=C, wherein the double bonds give rise to cis or trans isomers,
D¹ is C=O or S(=O)ₚ, wherein p is 0, 1 or 2,
D² is CHOH, O or S(=O)_{q}, wherein q is 0, 1 or 2,
B is oxygen or a group of the formula NR¹⁸, wherein R¹⁸ is hydrogen, an alkyl-, alkenyl-, alkynyl-, heteroalkyl-, aryl-, heteroaryl-, cycloalkyl-, alkylcycloalkyl-, heteroalkylcycloalkyl-, heterocycloalkyl-, aralkyl- or heteroarylalkyl-group and
R¹⁶ and R¹⁷ independently from each other represent hydrogen, C₁-C₄-alkyl or together are part of a cycloalkyl group with 3 or 4 ring atoms,
wherein one hydrogen atom of the compound of Formula (Epo) is replaced by the bond to linker L.

6. A compound according to claim 1, 2 or 3 having the following formula (II): 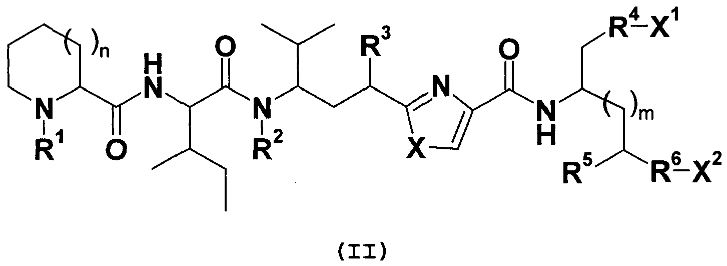 wherein
n = 0, 1, 2;
R¹ is an alkyl or a heteroalkyl group;
R² is hydrogen, an optionally substituted alkyl, alkenyl, alkinyl, heteroalkyl, cycloalkyl, heterocycloalkyl, alkylcycloalkyl, heteroalkylcycloalkyl, aryl, heteroaryl, aralkyl or heteroaralkyl group;
R³ is is hydrogen, OH, an optionally substituted alkyl, alkenyl, alkinyl, heteroalkyl, cycloalkyl, heterocycloalkyl, alkylcycloalkyl, heteroalkylcycloalkyl, aryl, heteroaryl, aralkyl or heteroaralkyl group;
X is O or S (especially S);
R⁴ is an optionally substituted arylene, heteroarylene, heterocycloalkylene, heteroalkylcycloalkylene, aralkylene or heteroaralkylene group;
R⁵ is hydrogen, an optionally substituted C₁-C₆ alkyl group, or an optionally substituted aryl or heteroaryl group;
R⁶ is -CO₂-, -CONH-, -CO-, -CONHNH-, -O-, -NH-, -S-, -SO-, -SO₂-, -OP(=O)O₂- or a branched or unbranched substituted or unsubstituted alkylene, cycloalkylene, heteroalkylene or heterocycloalkylene group;
m is 0, 1, 2 or 3;
X¹ is -L-Pep and X² is H or X¹ is H and X² is -L-Pep; and
L and Pep are as defined herein;
or a pharmacologically acceptable salt, a solvate, a hydrate or a pharmacologically acceptable formulation thereof.

7. A compound according to claim 1, 2 or 3 having the following formula (III): 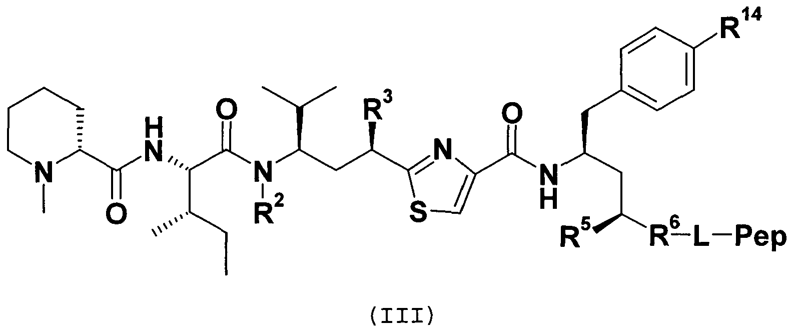 wherein
R² is C₁-C₆ alkyl (especially CH₃, ethyl, propyl, isopropyl, isobutyl, n-pentyl or n-hexyl), CH₂Ph, CH₂OCH₂CH(CH₃)₂, CH₂OCOCHC₁-C₆-Alkyl, CH₂OCOCH₂Ph or CH₂OCOPh;
R³ is H, OAc or OPr;
R⁵ i s CH₃ or H;
R⁶ is -COO-, -CONH-, -CONHNH-, -O-, -NH-, -CH₂O-, -CH₂NH- or -CH₂S-;
R¹⁴ is H, F, OH, NH₂, CH₃, OMe or Ph; and
L and Pep are as defined above;
or a pharmacologically acceptable salt, a solvate, a hydrate or a pharmacologically acceptable formulation thereof.

8. A pharmaceutical composition containing a compound according to anyone of Claims 1 to 7 and optionally one or more carriers and/or adjuvants.

9. A compound according to anyone of claims 1 to 7 or a pharmaceutical composition according to claim 8 for use in the treatment of cancer.


SEQUENCE LISTING

<110>  OntoChem GmbH

<120>  Receptor Ligand Linked Cytotoxic Molecules

<130>  20308

<160>  1

<170>  PatentIn version 3.5

<210>  1
<211>  36
<212>  PRT
<213>  Artificial Sequence

<220>
<223>  [F7, P34] NPY


<220>
<221>  PEPTIDE
<222>  (1)..(36)
<223>  [F7,P34]NPY

<400>  1

Tyr Pro Ser Lys Pro Asp Phe Pro Gly Glu Asp Ala Pro Ala Glu Asp
1               5                   10                  15


Leu Ala Arg Tyr Tyr Ser Ala Leu Arg His Tyr Ile Asn Leu Ile Thr
            20                  25                  30


Arg Pro Arg Tyr
        35